# EUROPEAN PATENT SPECIFICATION

(11) **EP 2 568 999 B1**
(45) Date of publication and mention of the grant of the patent: **11.07.2018**
(21) Application number: 11781106.7
(22) Date of filing: 10.05.2011
(51) Int. Cl.: A61K 38/22, A61K 38/16, A61P 21/02, A61P 21/00, A61K 38/35

(54) **ACTH FOR TREATMENT OF AMYOTROPHIC LATERAL SCLEROSIS**
ACTH ZUR BEHANDLUNG VON AMYOTROPHER LATERALSKLEROSE
ACTH POUR LE TRAITEMENT DE LA SCLÉROSE LATÉRALE AMYOTROPHIQUE

(30) Priority: 11.05.2010 US 333661 P
(43) Date of publication of application: 20.03.2013
(73) Proprietor: MALLINCKRODT ARD IP LIMITED, Blanchardstown (IE)
(72) Inventor: SOMERA-MOLINA, Kathleen, C., Huntley IL 60142 (US)
(74) Representative: Jones, Nicholas Andrew
(86) International application number: PCT/US2011/035831
(87) International publication number: WO 2011/143152

(56) References cited:
- WO-A1-93/00922
- WO-A2-2006/021814
- US-A1- 2005 009 847
- US-A1- 2005 026 937
- US-B2- 7 045 529
- GILBERT H. GLASER: 'Psychotic Reactions Induced by Corticotropin (ACTH) and Cortisone' PSYCHOSOMATIC MEDICINE vol. 15, no. 4, 01 July 1953, pages 280 - 291

## Description

### BACKGROUND OF THE INVENTION

Amyotrophic Lateral Sclerosis (ALS) is a fatal neurodegenerative disease that is characterized by progressive loss of motor neurons in the spinal cord, brainstem and/or the motor cortex. About 5-10% of ALS patient show familial traits; in more than 90% of patients, the disease is sporadic and does not show familial traits. The disease is fatal within about three years of diagnosis and fatality is generally due to atrophy of muscles necessary for breathing including the diaphragm.

### SUMMARY OF THE INVENTION

Described herein are methods of treatment of Amyotrophic Lateral Sclerosis (ALS) comprising administration of adrenocorticotropic hormone (ACTH), or ACTH-like compound, composition and/or preparation to an individual in need thereof. In some instances, ALS is associated with dysregulation of adrenal activity and/or abnormal secretion of ACTH. In some instances, normal levels of ACTH protect against loss of motor coordination and prevents degeneration of myelinated axons. In some instances, secretion of abnormal physiological levels of ACTH is associated with loss of motor neurons and/or motor function and/or muscle strength with subsequent manifestation of symptoms of ALS. Accordingly, administration of adrenocorticotropic hormone (ACTH), or ACTH-like compound, composition and/or preparation to an individual in need thereof, as described herein, provides neuroprotection, or in some instances, a neurotrophic effect, thereby alleviating symptoms of ALS. Further, administration of adrenocorticotropic hormone (ACTH), or ACTH-like compound, composition and/or preparation to an individual in need thereof, has an anti-inflammatory effect (e.g., reduction in release of neuro inflammatory cytokines), thereby alleviating symptoms of ALS.

Accordingly, the methods of treatment of ALS described herein may comprise administration of adrenocorticotropic hormone (ACTH) peptide, or fragment, analog, complex or aggregate thereof, or any combination thereof, to an individual in need thereof (e.g., an individual suffering from, suspected of having or predisposed to ALS) in doses and/or dosing regimens that allow for maintenance or restoration of the beneficial effects of ACTH, while reducing or reversing any detrimental effects caused by abnormal physiological levels of ACTH. The methods of treatment of ALS described herein may comprise administration of adrenocorticotropic hormone (ACTH) to an individual in need thereof (e.g., an individual suffering from, suspected of having or predisposed to ALS) in doses and/or dosing regimens such that physiological levels of ACTH in the individual are maintained, or rendered partially or substantially normal. The methods of treatment of ALS described herein may improve muscle action potential amplitudes and/or scores on functional rating tests such as the Amyotrophic Lateral Sclerosis Functional Rating Scale-revised (ALSFRSr) test.

The methods of treatment of ALS described herein may comprise administration of adrenocorticotropic hormone (ACTH) to an individual in need thereof (e.g., an individual suffering from, suspected of having or predisposed to ALS) in dosing regimens that are not continuous such as, for example, pulsed dosing regimens.

Detailed herein are methods of treating an individual diagnosed with, suspected of having, or predisposed to ALS comprising administration of adrenocorticotropic hormone (ACTH) peptide, or fragment, analog, complex or aggregate thereof, or any combination thereof, to an individual in need thereof, wherein the ACTH peptide or fragment, analog, complex or aggregate thereof, or any combination thereof, is administered as a first dose and one or more subsequent doses.

In the present invention, there is provided an adrenocorticotropic hormone (ACTH) peptide (ACTH₁₋₃₉ peptide) for use in delaying the progression of Amyotrophic Lateral Sclerosis (ALS) in an individual suspected of having, or predisposed to Amyotrophic Lateral Sclerosis (ALS), wherein the ACTH peptide is to be administered as a first dose and one or more subsequent doses.

In some embodiments of the present invention, the ALS is sporadic ALS. In some embodiments, the ALS is familial type ALS.

In some embodiments, the ACTH peptide is to be administered in early stage ALS upon onset of muscle weakness in the limbs and/or slurred and nasal speech.

In some embodiments, the ACTH peptide is to be administered upon detection of a mutation in the SOD1 gene.

In some embodiments, the ALS is associated with adrenal dysfunction.

In some embodiments, the first dose of ACTH peptide comprises a dose between about 10 IU and about 150 IU, and the one or more subsequent doses of ACTH peptide is administered about every day, about every 2 days, about every 5 days, about every week, about every two weeks, about every three weeks, about every month, about every two months, or any combination thereof.

In some embodiments, the first dose of ACTH peptide, comprises a dose between about 10 IU and about 100 IU, and the one or more subsequent doses of ACTH peptide are administered about every day, about every 2 days, about every 5 days, about every week, about every two weeks, about every three weeks, about every month, about every two months, or any combination thereof.

In some embodiments, the first dose of ACTH peptide comprises a dose between about 10 IU and about 150 IU, and the one or more subsequent doses of ACTH peptide are the same as the amount of the first dose.

In some embodiments, the first dose of ACTH peptide comprises a dose between about 10 IU and about 150 IU, and the one or more subsequent doses of ACTH peptide are between about 20% - 80% of the first dose.

In some embodiments, the first dose of ACTH peptide comprises a first dose of between about 10 IU and about 150 IU, and the one or more subsequent doses of ACTH peptide are between about 20% - 60% of the first dose.

In some embodiments, the first dose of ACTH peptide comprises a first dose of between about 10 IU and about 150 IU, and the one or more subsequent doses of ACTH peptide are between about 10 IU and about 80 IU.

In some embodiments, the doses of ACTH peptide, are to be administered every 2 days. In some embodiments, the doses of ACTH peptide are to be administered every 3 days. In some embodiments, the doses of ACTH peptide are to be administered every 4 days. In some embodiments, the doses of ACTH peptide are to be administered every 5 days. In some embodiments, the doses of ACTH peptide are to be administered every 6 days. In some embodiments, the doses of ACTH peptide are to be administered every 7 days.

The ACTH peptide may be administered to an individual in need thereof in an amount sufficient to provide plasma cortisol secretion levels between about 1.1 to about 4 times the plasma cortisol secretion levels of a normal individual at about 8 am.

The ACTH peptide may be administered to an individual in need thereof in an amount sufficient to provide plasma cortisol secretion levels between about 1.1 to about 4 times the plasma cortisol secretion levels at about 8 am of the individual prior to administration of the ACTH peptide.

In embodiments of the invention, the ACTH peptide is an ACTH₁₋₃₉ peptide having the formula:

H -Ser-Tyr-Ser-Met-Glu-His-Phe-Arg-Trp-Gly-
1 2 3 4 5 6 7 8 9 10
Lys-Pro-Val-Gly-Lys-Lys-Arg-Arg-Pro-Val-
11 12 13 14 15 16 17 18 19 20
Lys-Val-Try-Pro-Asp-Gly-Ala-Glu-Asp-Gln-
21 22 23 24 25 26 27 28 29 30
Leu-Ala-Glu-Ala-Phe-Pro-Leu-Glu-Phe-OH
31 32 33 34 35 36 37 38 39

In some of the embodiments described herein, the ACTH peptide is administered as an ACTH preparation. For example, in some embodiments, an ACTH preparation comprises an ACTH peptide and any other proteins and/or other substances that are present in a homogenized pituitary extract obtained from an appropriate animal source (e.g., a pig pituitary extract).

The ACTH peptide may be administered as a prodrug. In some embodiments, the ACTH peptide for the use detailed above is for administration as a modified release formulation. In some embodiments, the ACTH peptide for use as detailed above is for administration as an immediate release formulation.

In some embodiments, the ACTH peptide for the use detailed above, is for intramuscular administration. In some embodiments, the ACTH peptide for the use detailed above is for subcutaneous administration. In some embodiments, the ACTH peptide for the use detailed above is for oral administration.

In some embodiments, the ACTH peptide for use as detailed above is for intravenous administration. In some embodiments, the ACTH peptide for use as detailed above is for intrathecal administration.

In some embodiments, the ACTH peptide, is a porcine ACTH peptide.

In some embodiments, the ACTH peptide is a human ACTH peptide. In some embodiments, the ACTH peptide is a recombinant ACTH peptide.

In some embodiments, the ACTH peptide for the use detailed above is for administration in combination with a second therapeutic agent that treats symptoms and/or modifies the disease state. In some of such embodiments, the second therapeutic agent is selected from riluzole, ceftriaxone, methylcobalamine, Aeolus 10150, edaravone, hepatocyte growth factor (HGF), insulin growth factor (IGF), Atorvastatin, Lithium carbonate, Avanier 07-ACR-123 (Zenvia®), SB-509, Talampanel, Thalidomide, Arimoclomol, Olanzapine, KNS-760704, memantine, tamoxifen, ONO-2506PO, MCI-186, pioglitazone, ALS-357, creatine monohydrate, TCH346, Botulinum toxin type B, tauroursodeoxycholic acid, Dronabinol, coenzyme Q10, YAM80, Olesoxime, escitalopram (Lexapro®), sodium phenylbutyrate, ISIS 333611, granulocyte stimulating factor, neuronal growth factor (NGF), brain-derived neurotrophic factor (BDNF), neurotrophin 3 (NT3), basic fibroblast growth factor (bFGF), R(+) pramipexole dihydrochloride monohydrate, Sodium Valproate, AVP-923, sNN0029, Antithymocyte globulin, cyclosporin, corticosteroids, modafinil, or anti-CD40L, wherein the second therapeutic agent is to be administered sequentially or simultaneously.

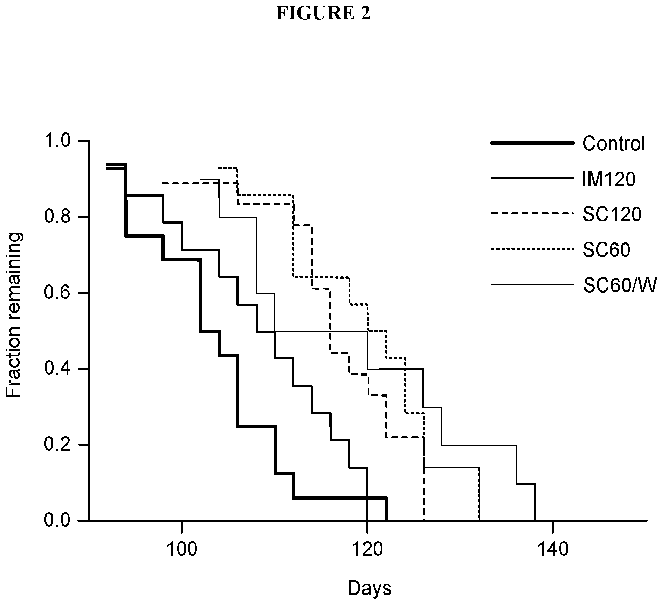

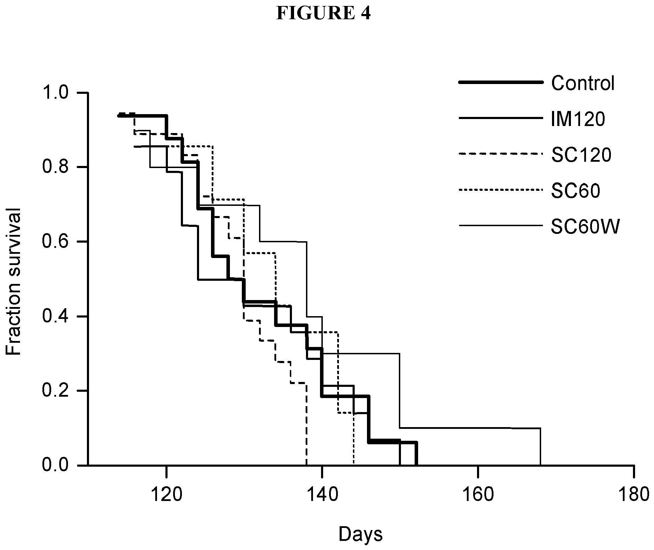

As described in the Examples section, including, Tables 1-2, and Figures 2-6, the methods described above delay progression of ALS thereby improving survival rate of individuals diagnosed with, suspected of having, or predisposed to ALS. In some embodiments, the methods described above reduce the degeneration of motor neurons associated with ALS. The methods and uses described above may increase muscle strength in individuals diagnosed with, suspected of having, or predisposed to ALS. The methods and uses described above may reduce or inhibit the release of pro-inflammatory cytokines associated with ALS. The methods and uses described above may increase the concentration of VEGF in spinal fluid. The methods and uses described above may reduce the concentration of mutant SOD1 aggregates in spinal fluid and/or motor neurons. The methods and uses described above may delay onset and/or severity of tremor in individuals diagnosed with, suspected of having, or predisposed to ALS. The methods and uses described above may delay progression of tremor to paralysis and/or spread of paralysis in individuals diagnosed with, suspected of having, or predisposed to ALS.

Provided herein, in some embodiments, is a pharmaceutical composition comprising an ACTH peptide for use in delaying progression of ALS and/or symptoms thereof.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

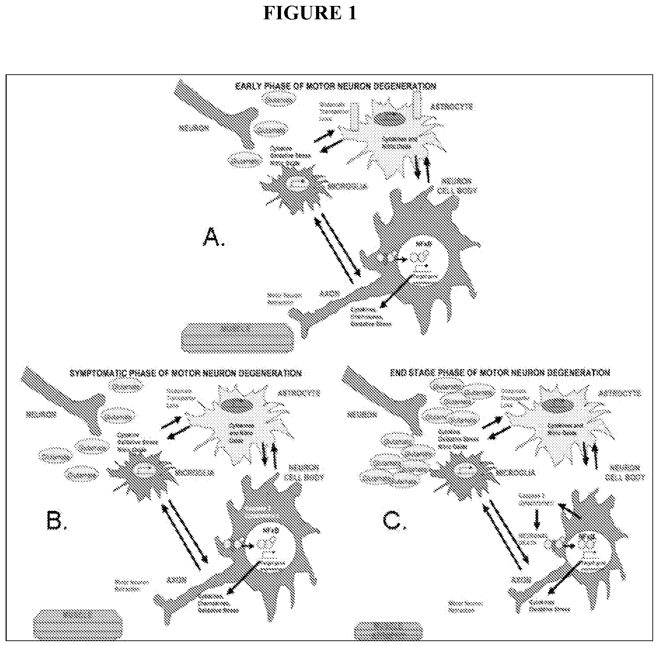

**Figure 1** illustrates a possible progression of motor neuron degeneration in ALS. (A) During early phase loss of glutamate transporters on astrocytes is observed and proinflammatory cytokines are produced. In some instance, such events lead to motor neuron axon retraction from muscle connections. (B) During symptomatic phase further loss of glutamate transporters leads to accumulation of glutamate neurotransmitters. Glial cells continue to produce proinflammatory cytokines and nitric oxide, a modulator of oxidative stress. As axons continue to retract, muscle loses source of energy, nutrition and stimulation. (C) Excitotoxicity and neuroinflammation contribute to motor neuron injury inducing release of mitochondrial cytochrome c, a signal initiating programmed cell death. During end stage ALS, motor neurons die and muscles shrink and deteriorate.
**Figure 2** illustrates the effect of intramuscular or subcutaneous ACTHAR® gel injections on onset of tremor in G93A SOD1 mice.
**Figure 3** illustrates the effect of intramuscular or subcutaneous ACTHAR® gel injections on paralysis proportions in G93A SOD1 mice.
**Figure 4** illustrates the effect of intramuscular or subcutaneous ACTHAR® gel injections on survival proportions in G93A SOD1 mice.
**Figure 5** illustrates measurement of SOD1 normalized by GAPDH activity from G93A SOD1 mice treated with intramuscular or subcutaneous ACTHAR® gel injections.
**Figure 6** illustrates staining of sections of anterior horn of the lumbar segment for SOD1, where segments were taken from G93A SOD1 mice treated with intramuscular or subcutaneous ACTHAR® gel injections.

### DETAILED DESCRIPTION OF THE INVENTION

ALS is a disease of motor neurons. Skeletal muscles are innervated by a group of neurons (lower motor neurons) located in the ventral horns of the spinal cord which project out the ventral roots to the muscle cells. These nerve cells are themselves innervated by the corticospinal tract or upper motor neurons that project from the motor cortex of the brain. In some instances, ALS is associated with a degeneration of the ventral horns of the spinal cord, as well as atrophy of the ventral roots. In the brain, motor neuron atrophy may be present in the frontal and/or the temporal lobes. In some instances, motor neuron degeneration in ALS is associated with adrenal dysregulation and/or abnormal levels of ACTH.

ACTH is a hormone that is secreted by the pituitary gland and is a part of the hypothalamus-pituitary-adrenal (HPA) axis that maintains the stress response and homeostasis in the body. In some instances ACTH plays a role in motor neuron function. Physiologically, the principal effects of ACTH are stimulation of the adrenal cortex with subsequent increased production of glucocorticosteroids and/or cortisol from the adrenal cortex. ACTH levels are tightly regulated in the body via a negative feedback loop wherein glucocorticosteroids suppress the release of corticotropin release hormone (CRH) from the pituitary and CRH-mediated release of ACTH. In some instances, cortisol helps restore homeostasis after stress. In some instances, changed patterns of serum cortisol levels are observed in connection with abnormal ACTH levels. In some instances, prolonged ACTH-mediated secretion of abnormal levels of cortisol (e.g., higher or lower levels of cortisol compared to cortisol levels in normal individuals) has detrimental effects. Thus, any perturbation in the levels of ACTH has profound physiological implications. The treatment of ALS presents unique challenges. In some instances, current treatment regimens are directed to alleviation of symptoms of ALS and improving quality of life for patients; however, such treatments lengthen survival only by a few months. Current treatment regimens do not address the unmet medical need for therapies that address the underlying etiology of ALS and/or delay the progression of the disease.

The peptide for use in the present invention reduces inflammation (e.g., neuroinflammation), thereby alleviating symptoms of ALS such as inflammation of motor neurons and/or musclature which affects, for example, the ability to swallow, or the ability to breathe. Disease progression may also be delayed. For example, where an individual suffering from ALS has paralysis on the left side of the body, administration of ACTH or ACTH-like compounds or preparations described herein delays further deterioration of the patient's condition, such as progression of paralysis to the right side of the patient's body.

Advantageously, the methods of treatment of ALS described herein may comprise administration of adrenocorticotropic hormone (ACTH) to an individual in need thereof in doses and/or dosing regimens such that, for example, any dysregulation in the HPA axis is remedied or partially remedied, thereby alleviating the symptoms of ALS and/or delaying progressive neurodegeneration associated with ALS, i.e., providing a neuroprotective effect. The doses and/or dosing regimens described herein may be designed to minimize any abrupt shifts in ACTH levels in an individual (e.g., a surge, or a drop in levels of ACTH). The methods of treatment of ALS described herein may delay, reduce or reverse damage to motor neurons, thereby allowing for long term survival of individuals diagnosed with, suspected of having, or predisposed to ALS.

The methods of treatment of ALS described herein may comprise administration of adrenocorticotropic hormone (ACTH) to an individual in need thereof in doses and/or dosing regimens such that, for example, the circadian rhythm and/or diurnal ACTH and/or cortisol levels of individuals diagnosed with, suspected of having, or predisposed to ALS trend towards the circadian rhythm and/or diurnal ACTH and/or cortisol levels of normal individuals, thereby alleviating the symptoms of ALS and/or delaying progressive neurodegeneration associated with ALS. The methods of treatment of ALS described herein may normalize ACTH levels and/or promote long term survival of an individual suffering from, suspected of having or predisposed to ALS compared to an individual who is not treated with ACTH or ACTH-like compounds and preparations described herein.

The methods of treatment of ALS described herein may comprise administration of adrenocorticotropic hormone (ACTH) or ACTH-like compounds or preparations to an individual pre-disposed to ALS (e.g., an individual having a family history of ALS, or a mutation in SOD1) in doses and/or dosing regimens such that onset of ALS is delayed.

### ALS

Amyotrophic Lateral Sclerosis (also called Motor Neuron Disease (MND), or Lou Gehrig's disease or Maladie de Charcot) is a progressive fatal neuromuscular disorder that is characterized by weakness, muscle wasting, and fasciculations (increased reflexes). Cognitive function is retained except where ALS is associated with dementia. The disease primarily affects motor neurons and is characterized by progressive degeneration of the motor neurons in the cerebral cortex, brainstem nuclei and anterior horns of the spinal cord. Individuals afflicted by the disease exhibit weakness of limbs, and difficulty in speech and swallowing. The weakness progresses to respiratory impairment and the disease is usually fatal and half of all patients die within about 3 years of onset of symptoms.

About 5-10% of ALS patients exhibit familial traits. About 20-30% of familial ALS patients exhibit a mutation in their copper/zinc superoxide dismutase (SOD1) gene. However, in greater than 90% of ALS patients, the disease is sporadic and the patients do not exhibit familial traits. In some instances, sporadic ALS patients exhibit Bunina bodies in motor neurons and are a pathological marker of ALS.

Neurons and glial cells (e.g., astrocytes) make up the neural framework in the brain. Astrocytes are responsible for maintenance of glutamate levels including the uptake and breakdown of glutamate. In some instances, the failure of astrocytes to sequester glutamate contributes to excess levels of glutamate and/or development of excitotoxicity. In some instances, ALS is associated with synaptic excitotoxicity that contributes to vulnerability of neurons to degeneration. In some instances, ALS patients exhibit higher levels of glutamate in spinal fluid and serum compared to levels of glutamate in spinal fluid and serum of individuals not suffering from ALS.

Glial cells play a role in maintenance of homeostasis in the brain. Activated glia secrete neurotrophic agents including neuronal growth factor (NGF), brain-derived neurotrophic factor (BDNF), neurotrophin 3 (NT3), basic fibroblast growth factor (bFGF) or the like. Glial cells also counteract neuroinflammation by secretion of anti-inflammatory molecules such as IL-10, Apolipoprotein E, IL-1 receptor antagonist or the like. Thus glial activation protects the brain from the effects of stress or injury. Aberrant glial activation is accompanied by overproduction of pro-inflammatory cytokines, chemokines and reactive oxygen species. In some instances, aberrant glial activation is caused by excess glutamate. In some instances, pro-inflammatory cytokines and/or oxidative stress propagate the cycle of chronic glial activation. In some instances, ALS patients show increased levels of pro-inflammatory cytokines in spinal cord tissue and Cerebrospinal fluid (CSF) compared to controls.

Familial ALS is associated with genetic mutations in the Superoxide Dismutase 1 gene (SOD1). Mutations in SOD1 are "gain of function" mutations. In some instances, defects in SOD1 cause accumulation of free radicals. In some instances, familial ALS patients with mutations in the SOD1 gene exhibit decreased levels of VEGF in the cerebrospinal fluid compared to controls. In some instances, mutations in the SOD1 gene are associated with decreased expression of VEGF receptors on the surface of motor neurons. In some instances, under-expression of VEGF receptors is associated with degeneration of motor neurons.

ALS is a chronic disease, often having an asymptomatic phase spanning 4-5 decades. During the asymptomatic phase, peripheral motor axons are maintained by monocyte populations. The onset of ALS is a non-symptomatic stage when there is retraction of motor axons from their synapses onto muscles. In some instances, neuroinflammation and/or oxidative stress in muscles and/or mitochondrial dysfunction and/or glutamate excitotoxicity and/or reduced expression of VEGF receptors is associated with onset of ALS. In some instances, mutations in SOD1 gene and/or aberrant serum levels of ACTH and/or cortisol are associated with onset of ALS. During the symptomatic phase of ALS, unknown mechanisms result in deleterious immune response with subsequent neuroinflammation and neurodegeneration. The symptomatic phase of ALS is characterized by damage to microglia and astrocytes, loss of muscle strength and slurred speech. In the final stages of the disease, there is paralysis and muscle atrophy. In some embodiments, therapeutic intervention at onset of disease, or in early stages of disease, includes modulating abnormal glutamate metabolism associated with astrocytes. In some embodiments, therapeutic intervention in later stages of disease includes modulating excitoxicity associated with astrocytes and/or modulating aberrant activation of glial cells, thereby delaying progression of disease. In some instances, therapeutic intervention in ALS includes inducing overexpression of VEGF, thereby reducing or delaying neurodegeneration.

### ACTH

Uses falling outside the claims are provided for reference only.

ACTH is a 39 amino acid peptide hormone secreted by the anterior pituitary gland. ACTH is secreted from the anterior pituitary in response to corticotropin-releasing hormone (CRH) that is secreted from the hypothalamus. The release of ACTH stimulates the adrenal cortex with subsequent increased production of glucocorticosteroids and/or cortisol from the adrenal cortex.

ACTH is synthesized from a precursor polypeptide pre-pro-opiomelanocortin (pre-POMC). The removal of the signal peptide during translation produces a 267 amino acid polypeptide POMC. POMC undergoes a series of post-translational modifications to yield various polypeptide fragments including and not limited to ACTH, β-lipotropin, γ-lipotropin, α, β, γ - Melanocyte Stimulating Hormone (MSH) and β-endorphin. POMC, ACTH and β-lipotropin are also secreted from the pituitary gland in response to the hormone corticotropin-releasing hormone (CRH). In some embodiments, the first 13 amino acids of ACTH₁₋₃₉ are cleaved to form α-melanocyte-stimulating hormone (a-MSH).

In some instances, multiple hypothalamic, pituitary, and peripheral factors regulate stress-mediated or inflammation-induced POMC expression and/or ACTH secretion. Essential cellular functions maintaining metabolic and neuroendocrine control require a homeostatic, non-stressed pattern of ACTH and glucocorticoid secretion. ACTH secretion is characterized by both circadian periodicity and ultradian pulsatility that is generated by CRH release and is also influenced by peripheral corticosteroids. Thus, ACTH secretion peaks at about before 7 am and nadir adrenal steroid secretion occurs between about 11 pm and 3 am, with periodic secretory bursts occurring throughout the day. Serum cortisol levels also exhibit a similar pattern of circadian periodicity. These rhythms are further reinforced by visual cues and the light-dark cycle. In some instances, stress results in increased ACTH pulse amplitude. In some instances, ALS is associated with abnormality in the circadian periodicity and ultradian pulsatility of ACTH and/or cortisol levels in the body.

In some instances, an abnormality in ACTH levels is associated with inflammation (e.g., increased release of pro-inflammatory cytokines). In some instances, an abnormality in ACTH levels is associated with reduced VEGF secretion. In some instances, reduced VEGF secretion is associated with reduced growth of new blood vessels and inadequate oxygen supply to tissues (e.g., neurons and/or muscles).

In some instances, ACTH promotes axonal regeneration. In some instances, ACTH acts as a neurotrophic factor. In some instances, endogenous ACTH increases muscle action potential. In some instances, ACTH levels in the body increase upon activation of certain glutamate receptors such as the NMDA receptors. In some other instances, excitotoxicty increases or decreases secretion of ACTH.

In some instances, ACTH regulates astrocyte response by binding to melanocortin receptors. In some instances, ACTH suppresses neuroinflammation associated with ALS by binding to melanocortin receptors. In some instances, ACTH regulates migration of inflammatory cells and maintains the integrity of the blood brain barrier.

### Definitions

The term "ACTH", in some embodiments, includes corticotropin, adrenocorticotropic hormone and Tetracosactide. In some embodiments, the term ACTH includes a 39 amino acid peptide hormone secreted by the anterior pituitary gland. The term "ACTH" may also include any ACTH peptide, any ACTH fragment, or any ACTH preparation as described herein. The term ACTH includes, in some embodiments, ACTH from any source including human ACTH, mouse ACTH, rat ACTH, porcine ACTH, sheep ACTH, bovine ACTH, rabbit ACTH or any other source of ACTH. In further embodiments, the term ACTH includes humanized and/or recombinant forms of ACTH and synthetic forms of ACTH.

The term "ACTH peptide" refers to ACTH₁₋₃₉ peptide of structure:

H -Ser-Tyr-Ser-Met-Glu-His-Phe-Arg-Trp-Gly-
1 2 3 4 5 6 7 8 9 1 0
Lys-Pro-Val-Gly-Lys-Lys-Arg-Arg-Pro-Val-
11 12 13 14 15 16 17 18 19 20
Lys-Val-Try-Pro-Asp-Gly-Ala-Glu-Asp-Gln-
21 22 23 24 25 26 27 28 29 30
Leu-Ala-Glu-Ala-Phe-Pro-Leu-Glu-Phe-OH
31 32 33 34 35 36 37 38 39.

The term "ACTH peptide homolog" includes ACTH peptide or peptide fragments or ACTH-like compounds with about 40%, about 45%, about 50%, about 55%, about 60%, about 65%, about 70%, about 75%, about 80%, about 85%, about 90%, or about 95% homology with ACTH₁₋₃₉.

The term "ACTH aggregate" refers to a physical grouping of peptides which may comprise ACTH peptide, or fragments, analogs or homologs thereof. Such an aggregate may comprise hydrogen-bonded molecules and/or molecules held by bridging interactions via, for example, a salt bridge or a metal ion.

The term "ACTH complex" refers to ACTH or fragments or analogs thereof that are optionally complexed with other proteins (e.g., Bovine Serum Albumin), or metal ions, or charged polymers (e.g., polylysine), or fragments, homologs or anlogs of ACTH, or any other suitable complexes that retain the functional characteristics of ACTH or ACTH fragments or analogs thereof and/or allow for formulation of ACTH or ACTH fragments or analogs thereof into suitable dosage forms.

In some embodiments, ACTH is an ACTH preparation. As used herein, "ACTH preparation" refers to a mixture containing ACTH peptide and/or other peptide fragments and/or other proteins and/or other substances that together form a composition that is suitable for any methods and/or dosing regimen described herein. In some of such embodiments, ACTH is obtained from a homogenized pituitary extract of an appropriate animal (e.g., pituitary extract of a pig). Any suitable method is used to obtain a homogenized pituitary extract. In some of such embodiments, a homogenized pituitary extract includes ACTH peptide and/or other peptide fragments and/or other proteins and/or other substances that are contemplated as being part of the ACTH preparation that is compatible with any method described herein.

The term "ACTH analog" or "analog of ACTH" refers to any compounds in which one or more atoms, functional groups, or substructures or amino acids in ACTH or fragments of ACTH have been replaced with different atoms, groups, or substructures or amino acids while retaining the functional activity of ACTH or fragments of ACTH. In some embodiments, an ACTH analog is a peptide fragment of ACTH₁₋₃₉ peptide that retains biological activity of ACTH, or in other words, has ACTH-like activity. One example of an ACTH analog is ACTH₄₋₉ peptide analog (ORG-2766) of formula:

Met-Glu-His-Phe-D-Lys-Phe-OH

or a fragment, complex, aggregate, or analog thereof, or any combination thereof.

An ACTH analog is a compound in which one or more amino acids in ACTH, or homolog or fragment thereof is conservatively modified or substituted with another amino acid such that the modification does not impact the ACTH-like activity. As to amino acid substitutions, individual substitutions, deletions or additions to a peptide sequence which alters, adds or deletes a single natural and non-natural amino acid or a small percentage of natural and non-natural amino acids in the encoded sequence is a "conservatively modified analog" where the alteration results in the deletion of an amino acid, addition of an amino acid, or substitution of a natural and non-natural amino acid with a chemically similar amino acid, while retaining the biological activity of the ACTH peptide or fragment thereof. Conservative substitution tables providing functionally similar natural amino acids are well known in the art. For example, the following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)
(*See,* e.g., Creighton, Proteins:Structures and Molecular Properties, W H Freeman & Co.; 2nd edition (December 1993)). In some embodiments, an ACTH analog has between 1-5 additional amino acid residues attached to the start or end of ACTH₁₋₃₉ peptide.

The term "ACTH fragment" includes any portion of the ACTH peptide ACTH₁₋₃₉. Examples of synthetic forms and/or fragments of ACTH include and are not limited to ACTH₁₋₂₄ peptide having the formula: 
H -Ser-Tyr-Ser-Met-Glu-His-Phe-Arg-Trp-Gly-
1 2 3 4 5 6 7 8 9 1 0
Lys-Pro-Val-Gly-Lys-Lys-Arg-Arg-Pro-Val-
11 12 13 14 15 16 17 18 19 20
Lys-Val-Try-Pro
21 22 23 24
 or a fragment, complex, aggregate, or analog thereof, or any combination thereof,

ACTH₁₋₁₇ peptide having the formula: 
H -Ser-Tyr-Ser-Met-Glu-His-Phe-Arg-Trp-Gly-
1 2 3 4 5 6 7 8 9 1 0
Lys-Pro-Val-Gly-Lys-Lys-Arg-
11 12 13 14 15 16 17
 or a fragment, complex, aggregate, or analog thereof, or any combination thereof, or

ACTH₄₋₁₀ peptide (ORG-066) of formula: 
Met-Glu-His-Phe-Arg-Trp-Gly
4 5 6 7 8 9 1 0
 or a fragment, complex, aggregate, or analog thereof, or any combination thereof.

The term ACTH fragment also includes alpha-MSH (ACTH₁₋₁₃) and d-alpha-MSH

H -Ser-Tyr-Ser-Met-Glu-His-Phe-Arg-Trp-Gly-
1 2 3 4 5 6 7 8 9 1 0
Lys-Pro-Val
11 12 13 .

Any ACTH peptide, fragment, complex, aggregate, or analog or homolog thereof described above or below retains ACTH-like activity. As used herein, "ACTH-like activity" , in some embodiments, may refer to activity of ACTH₁₋₃₉ peptide which is responsible for (1) steroidogenesis via interaction at, for example, melanocortin receptor MCR2, and/or (2) neuroprotective and/or anti-inflammatory activity mediated via interaction of ACTH, or fragment, anolog or homolog thereof at, for example, melanocotin receptors 1 and 3 (MCR1 and MCR3). Thus ACTH-like activity for a fragment arises from different domains of the ACTH₁₋₃₉ peptide. Accordingly, in one embodiment, ACTH-like activity at, for example, MCR2, resides in residues 14-39 of the ACTH₁₋₃₉ peptide. In a different embodiment, ACTH-like activity at, for example, MCR1 and MCR3, resides in residues 6-9 of the ACTH₁₋₃₉ peptide.

The term "ACTH peptide, or fragment, analog, complex or aggregate thereof' includes, in addition to embodiments described above or below, a peptide of formula

Ac-Ser-Tyr-Ser-Met-Glu-His-Phe-Arg-Trp-Gly-Lys-Pro-Val

or a fragment, complex, aggregate, or analog thereof, or any combination thereof, or a peptide fragment of formula:

H-Ser-Tyr-Ser-Met-Glu-His-Phe-Arg-Trp-Gly-Lys-Pro-Val

or a fragment, complex, aggregate, or analog thereof, or any combination thereof, or a peptide fragment of formula:

D-Ala-Gln-Tyr-Phe-Arg-Trp-Gly-NH₂

or a fragment, complex, aggregate, or analog thereof, or any combination thereof.

The term "ACTH peptide, or fragment, analog, complex or aggregate thereof' also includes pre-POMC, POMC, β-lipotropin, γ-lipotropin, Melanocyte Stimulating Hormone (a-MSH, β-MSH, γ-MSH), β-endorphin, or any other polypeptide fragment that is a post-translational product of the POMC gene. POMC genes for various species are found in the NCBI GenBank including and not limited to human POMC transcript variant 1, mRNA, (NCBI Accession number NM_001035256), human POMC transcript variant 2, mRNA, (NCBI Accession number NM_000939), swine pro-opiomelanocortin, mRNA (NCI Accession number S73519), swine proopiomelanocortin protein (POMC) gene (NCBI Accession number EU184858), rat proopiomelanocortin (POMC) gene (NCBI Accession number K01877). Other examples of POMC genes include, for example, catfish POMC gene described in Animal Genetics, 2005, 36, 160-190. Melanocortin peptides, including ACTH and alpha, beta and gamma MSH derive from post-translational modification of POMC. A number of melanocortin peptides share an invariant sequence of four amino acids, His-Phe-Arg-Trp, which also correspond to residues 6-9 of ACTH and alpha-MSH. Accordingly, also contemplated within the scope of embodiments presented herein, is the use of amino acid sequences that correspond to alpha MSH, beta MSH or gamma MSH. *See* Catania et al., Pharmacol. Rev. 2004, 56: 1-29. The term "ACTH peptide, or fragment, analog, complex or aggregate thereof' also includes, an antibody that binds to melanocortin receptors and possess ACTH-like activity.

The term "ACTH peptide, or fragment, analog, complex or aggregate thereof' also includes synthetic preparations of ACTH that are commercially available including and not limited to ACTHAR® powder for injection or gel, Synacthen® or Adrenomone®. Examples of commercially available ACTH peptides that are compatible with the methods described herein include and are not limited to Adrenocorticotropic Hormone (ACTH) (1-10) (human), Adrenocorticotropic Hormone (ACTH) (1-13) (human), Adrenocorticotropic Hormone (ACTH) (1-16) (human), Adrenocorticotropic Hormone (ACTH) (1-17) (human), Adrenocorticotropic Hormone (ACTH) (1-24) (human), Adrenocorticotropic Hormone (ACTH) (1-39) (human), Adrenocorticotropic Hormone (ACTH) (1-39) (rat), Adrenocorticotropic Hormone (ACTH) (18-39) (human), Adrenocorticotropic Hormone (ACTH) (4-10) (human), Adrenocorticotropic Hormone (ACTH) (1-4), Adrenocorticotropic Hormone (ACTH) (1-14) available from, for example, GenScript.

The term "prodrug" refers to a precursor molecule that is a derivative of ACTH or ACTH fragments or analogs thereof that is suitable for incorporation in any dosage form described herein. A "prodrug" refers to a precursor compound that is converted into active compound *in vivo.* Prodrugs are often useful because, in some situations, they may be easier to administer than the parent drug. They may, for instance, be bioavailable by oral administration whereas the parent is not. The prodrug may also have improved solubility in pharmaceutical compositions over the parent drug. Prodrugs may facilitate transmittal across a cell membrane where water solubility is detrimental to mobility but which then is metabolically hydrolyzed to the carboxylic acid, the active entity, once inside the cell where water-solubility is beneficial. As non-limiting examples, a prodrug of ACTH or fragment of analog thereof is metabolically stable and is not degraded in the stomach.

Prodrugs are generally drug precursors that, following administration to a subject and subsequent absorption, are converted to an active, or a more active species via some process, such as conversion by a metabolic pathway. Some prodrugs have a chemical group present on the prodrug that renders it less active and/or less labile and/or confers solubility or some other property to the drug. Once the chemical group has been cleaved and/or modified from the prodrug the active drug is generated. A prodrug of ACTH or fragment or analog thereof may be an alkyl ester of the parent compound such as, for example, methyl ester, ethyl ester, n-propyl ester, iso-propyl ester, n-butyl ester, sec-butyl ester, tert-butyl ester or any other ester.

### Methods

Detailed herein are methods of treating ALS comprising administration of ACTH to individuals in need thereof. The methods of treatment of ALS described herein allow for early intervention upon detection of loss of muscle strength and/or slurred speech and prior to onset of twitches/paralysis. Upon detection of a mutation in SOD1, the methods of treatment of ALS described herein may allow for prophylactic administration of ACTH in familial ALS patients and/or patients who are pre-disposed to ALS and allow for delayed onset of disease or for slowing down progression of disease.

In some instances, secretion of abnormal physiological levels of ACTH (e.g., lower levels or higher physiological levels of ACTH compared to normal physiological levels of ACTH) is associated with loss of motor neurons and/or motor function and/or muscle strength with subsequent manifestation of symptoms of ALS. Accordingly, administration of ACTH may allow for correction of abnormal physiological levels of ACTH, thereby alleviating symptoms of ALS and/or slowing down disease progression.

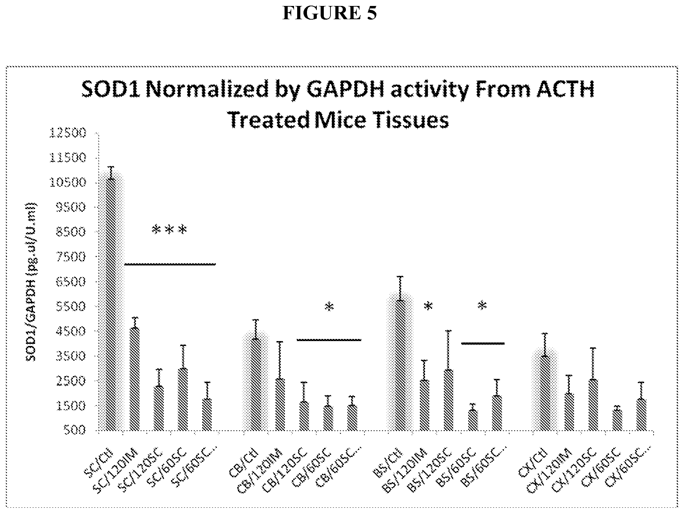

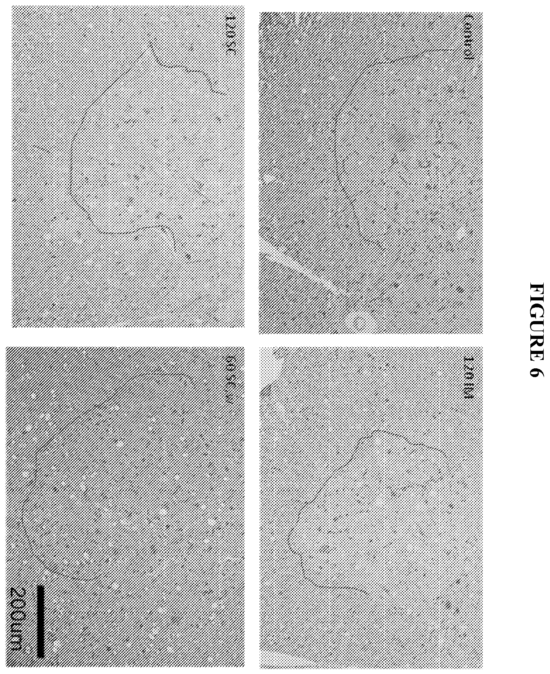

Example 3, Table 2, Figure 2, Figure 3 and Figure 4 show that animals treated with ACTHAR® gel show a delay in onset of tremor, and/or a trend for reduced paralysis and/or a trend for increased survival. Figure 5 shows decreased expression and deposition of SOD1 protein in treated animals in various brain and spinal cord tissues. Figure 6 shows staining of anterior horn of the lumbar segment of the spinal cord illustrating decreased expression and deposition of SOD1 protein in treated animals.

Described herein are methods of treating Amyotrophic Lateral Sclerosis comprising administration of adrenocorticotropic hormone (ACTH) peptide to an individual diagnosed with, suspected of having, or predisposed to ALS, wherein the ACTH is administered as a first dose and one or more subsequent doses. In some of such embodiments, the individual has a mutation in the SOD1 gene.

Described herein are methods of treating or reducing paralysis and/or spread of paralysis associated with Amyotrophic Lateral Sclerosis comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of alleviating tremor associated with Amyotrophic Lateral Sclerosis comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of delaying or slowing down the progression of Amyotrophic Lateral Sclerosis comprising administration of adrenocorticotropic hormone (ACTH) peptide to an individual diagnosed with, suspected of having, or predisposed to ALS, wherein the ACTH peptide is administered as a first dose and one or more subsequent doses. Administration of a dosing regimen of adrenocorticotropic hormone (ACTH) peptide, as described herein, to an individual diagnosed with, suspected of having, or predisposed to ALS may reduce or inhibit axonal demyelination, thereby delaying progression of ALS.

Described herein are methods of reducing the degeneration of motor neurons associated with ALS comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of increasing muscle strength in an individual diagnosed with, suspected of having, or predisposed to ALS comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of suppressing the release of pro-inflammatory cytokines associated with ALS comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of increasing the concentration of VEGF in spinal fluid in an individual diagnosed with, suspected of having, or predisposed to ALS comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of reducing the concentration of mutant SOD1 aggregates in spinal fluid and/or motor neurons in an individual diagnosed with, suspected of having, or predisposed to ALS comprising administration of adrenocorticotropic hormone (ACTH) peptide to an individual diagnosed with, suspected of having, or predisposed to ALS, wherein the ACTH peptide is administered as a first dose and one or more subsequent doses.

Described herein are methods of reducing glutamate excitotoxicity (e.g., by reducing glutamate levels in the CSF) in an individual diagnosed with, suspected of having, or predisposed to ALS comprising administration of adrenocorticotropic hormone (ACTH) peptide to an individual diagnosed with, suspected of having, or predisposed to ALS, wherein the ACTH peptide is administered as a first dose and one or more subsequent doses.

In any of the methods described above or below, the adrenocorticotropic hormone (ACTH) peptide suitable for the methods of treatment of ALS provided herein may be ACTH₄₋₉. In any of the methods described above or below, the adrenocorticotropic hormone (ACTH) peptide suitable for the methods of treatment of ALS provided herein may be ACTH₄₋₁₀. In any of the methods described above or below, the adrenocorticotropic hormone (ACTH) suitable for the methods of treatment of ALS provided herein may be ACTH₁₋₁₇. In any of the methods described above or below, the adrenocorticotropic hormone (ACTH) peptide suitable for the methods of treatment of ALS provided herein may be ACTH₁₋₁₃ (alpha-MSH or d-alpha MSH). In any of the methods described above or below, the adrenocorticotropic hormone (ACTH) peptide suitable for the methods of treatment of ALS provided herein may be ACTH₁₋₂₄. In any of the methods described above or below, the adrenocorticotropic hormone (ACTH) peptide suitable for the methods of treatment of ALS provided herein may be any synthetic commercial preparation described herein or any POMC derived molecule described herein, and even more preferably ACTH₁₋₃₉. In any of the methods described above, the adrenocorticotropic hormone (ACTH) peptide suitable for the methods described herein may be an ACTH₁₋₃₉ preparation (e.g., ACTHAR®).

An ACTH preparation suitable for methods of treatment described herein may comprise a mixture of ACTH₁₋₃₉ and one or more POMC-derived molecules described herein.

Administration of a dosing regimen of adrenocorticotropic hormone (ACTH) peptide to an individual diagnosed with, suspected of having, or predisposed to ALS may maintain ACTH levels (e.g., maintain ACTH levels without any further decline or increase) in the individual, or changes ACTH levels to partially normal or substantially normal levels. As used herein, a "change to substantially normal ACTH levels" refers to a change in physiological levels of ACTH levels in an individual suffering from, suspected of having, or pre-disposed to ALS to levels that are substantially the same as the levels of ACTH in a normal individual when measured at about the same time (e.g., at 8 am). As used herein, substantially the same means, for example, about 90% to about 110% of the measured ACTH levels in a normal individual when measured at about the same time (e.g., at 8 am). In other embodiments, substantially the same means, for example, about 80% to about 120% of the measured ACTH levels in a normal individual when measured at the about same time (e.g., at 8 am). As used herein, "change to partially normal level of ACTH" refers to any change in ACTH levels in an individual suffering from, suspected of having, or pre-disposed to ALS that trends towards ACTH levels of a normal individual when measured at about the same time (e.g., at 8 am). As used herein "partially normal ACTH level" is, for example, ± about 25%, ± about 35%, ± about 45%, ± about 55%, ± about 65%, or ± about 75% of the measured ACTH level of a normal individual when measured at the about same time (e.g., at 8 am).

Administration of a dosing regimen of adrenocorticotropic hormone (ACTH) peptide, as described herein, to an individual diagnosed with, suspected of having, or predisposed to ALS may maintain cortisol levels (e.g., maintain cortisol levels without any further decline or increase) in the individual, or changes cortisol levels to partially normal or substantially normal levels. As used herein, a "change to substantially normal cortisol levels" refers to a change in physiological levels of cortisol levels in an individual suffering from, suspected of having, or pre-disposed to ALS to levels that are substantially the same as the levels of cortisol in a normal individual when measured at about the same time (e.g., at 8 am). As used herein, substantially the same means, for example, about 90% to about 110% of the measured cortisol levels in a normal individual when measured at about the same time (e.g., at 8 am). In other embodiments, substantially the same means, for example, about 80% to about 120% of the measured cortisol levels in a normal individual when measured at the about same time (e.g., at 8 am). As used herein, "change to partially normal level of cortisol" refers to any change in cortisol levels in an individual suffering from, suspected of having, or pre-disposed to ALS that trends towards cortisol levels of a normal individual when measured at about the same time (e.g., at 8 am). As used herein "partially normal cortisol level" is, for example, ± about 25%, ± about 35%, ± about 45%, ± about 55%, ± about 65%, or ± about 75% of the measured cortisol level of a normal individual when measured at the about same time (e.g., at 8 am).

Administration of a dosing regimen of adrenocorticotropic hormone (ACTH) peptide, as described herein, to an individual diagnosed with, suspected of having, or predisposed to ALS may maintain VEGF receptor expression levels on motor neurons (e.g., maintain VEGF receptor expression levels on motor neurons levels without any further decline or increase) in the individual, or changes VEGF receptor expression levels on motor neurons to partially normal or substantially normal levels. As used herein, a "change to substantially normal ACTH levels" refers to a change in physiological levels of VEGF receptor expression on motor neurons levels in an individual suffering from, suspected of having, or pre-disposed to ALS to levels that are substantially the same as the levels of VEGF receptor expression on motor neurons in a normal individual when measured at about the same time (e.g., at 8 am). As used herein, substantially the same means, for example, about 90% to about 110% of the measured VEGF receptor expression levels on motor neurons in a normal individual when measured at about the same time (e.g., at 8 am). In other embodiments, substantially the same means, for example, about 80% to about 120% of the measured VEGF receptor expression levels on motor neurons in a normal individual when measured at the about same time (e.g., at 8 am). As used herein, "change to partially normal level of VEGF receptor expression on motor neurons" refers to any change in VEGF receptor expression levels on motor neurons levels in an individual suffering from, suspected of having, or pre-disposed to ALS that trends towards VEGF receptor expression levels on motor neurons of a normal individual when measured at about the same time (e.g., at 8 am). As used herein "partially normal VEGF receptor expression levels on motor neurons level" is, for example, ± about 25%, ± about 35%, ± about 45%, ± about 55%, ± about 65%, or ± about 75% of the measured VEGF receptor expression levels on motor neurons of a normal individual when measured at the about same time (e.g., at 8 am).

Administration of a dosing regimen of adrenocorticotropic hormone (ACTH) peptide, as described herein, to an individual diagnosed with, suspected of having, or predisposed to ALS may maintain glutamate levels in the CSF (e.g., maintain glutamate levels in the CSF without any further decline or increase) in the individual, or changes glutamate levels in the CSF to partially normal or substantially normal levels. As used herein, a "change to substantially normal glutamate levels in the CSF" refers to a change in physiological levels of glutamate in the CSF of an individual suffering from, suspected of having, or pre-disposed to ALS to levels that are substantially the same as the levels of glutamate in the CSF of a normal individual when measured at about the same time (e.g., at 8 am). As used herein, substantially the same means, for example, about 90% to about 110% of the measured glutamate levels in the CSF of a normal individual when measured at about the same time (e.g., at 8 am). In other embodiments, substantially the same means, for example, about 80% to about 120% of the measured glutamate levels in the CSF of a normal individual when measured at the about same time (e.g., at 8 am). As used herein, "change to partially normal level of glutamate levels in the CSF" refers to any change in glutamate levels in the CSF of an individual suffering from, suspected of having, or pre-disposed to ALS that trends towards glutamate levels in the CSF of a normal individual when measured at about the same time (e.g., at 8 am). As used herein "partially normal glutamate levels in the CSF" is, for example, ± about 25%, ± about 35%, ± about 45%, ± about 55%, ± about 65%, or ± about 75% of the measured glutamate levels in the CSF of a normal individual when measured at the about same time (e.g., at 8 am).

Administration of a dosing regimen of adrenocorticotropic hormone (ACTH) peptide, as described herein, to an individual diagnosed with, suspected of having, or predisposed to ALS may maintain SOD1 load in the CSF (e.g., maintain SOD1 load in the CSF without any further decline or increase) in the individual, or changes SOD1 load in the CSF to partially normal or substantially normal levels. As used herein, a "change to substantially normal SOD1 load in the CSF" refers to a change in physiological levels of SOD1 load in the CSF of an individual suffering from, suspected of having, or pre-disposed to ALS to levels that are substantially the same as the SOD1 load in the CSF of a normal individual when measured at about the same time (e.g., at 8 am). As used herein, substantially the same means, for example, about 90% to about 110% of the measured SOD1 load in the CSF of a normal individual when measured at about the same time (e.g., at 8 am). In other embodiments, substantially the same means, for example, about 80% to about 120% of the measured SOD1 load in the CSF of a normal individual when measured at the about same time (e.g., at 8 am). As used herein, "change to partially normal level of SOD1 load in the CSF" refers to any change in SOD1 load in the CSF of an individual suffering from, suspected of having, or pre-disposed to ALS that trends towards SOD1 load in the CSF of a normal individual when measured at about the same time (e.g., at 8 am). As used herein "partially normal SOD1 load in the CSF" is, for example, ± about 25%, ± about 35%, ± about 45%, ± about 55%, ± about 65%, or ± about 75% of the measured SOD1 load in the CSF of a normal individual when measured at the about same time (e.g., at 8 am).

### Determination of therapeutic effect

Certain endpoints are used to determine therapeutic efficacy of administration of a dosing regimen of adrenocorticotropic hormone (ACTH) peptide or fragment, analog, complex or aggregate thereof, or any combination thereof, as described herein, to an individual diagnosed with, suspected of having, or predisposed to ALS. Examples of such endpoints include reduction in rate of weight loss, delay in onset or spread of paralysis, extension of survival, number of motor neurons in spinal cord, reduction in inflammation of the spinal cord, reduction in rate of loss of motor neuron cell bodies, reduction in macrophages in the sciatic nerve, reduction in expression of certain genes (e.g., genes described by Lincecum et al. in Nature Genetics, Advanced Online Publication March 28, 2010), reduction in astrocytosis and microgliosis, or any other detectable and/or measurable endpoint, or any combination thereof. In some instances, co-stimulatory genes are activated in certain patient populations (e.g., co-stimulatory genes and/or pathways described in Lincecum et al. in Nature Genetics, Advanced Online Publication March 28, 2010). Expression of upregulated genes in the co-stimulatory pathways may serve as biomarker for disease progression and/or effect of any therapy described herein.

Accordingly, in some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of reduction in rate of weight loss.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of delay in onset of paralysis.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of extension of survival.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of number of motor neurons in spinal cord.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of reduction in inflammation of the spinal cord.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of reduction in rate of loss of motor neuron cell bodies.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of severity and/or duration of tremor. In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of severity and/or duration of paralysis, and/or evaluation of spread of paralysis.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of muscle or motor performance. Examples of such tests include, for example the rotarod performance test in mice: Motor coordination is assessed by measuring the length of time for which mice remained on the rotating rod (16 r.p.m.). The Postural reflex test is conducted essentially as described by Bederson et al., Stroke, 1986, 17, 472-476 to examine the strength of the forelimbs in mice. In the screen test which serves as an indicator of general muscle strength, an animal is placed on a horizontally positioned screen with grids. The screen is then rotated to the vertical position and the length of time before the animal falls off is measured.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of reduction in macrophages in the sciatic nerve.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of reduction in expression of certain genes.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of reduction in astrocytosis and microgliosis.

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of activation of co-stimulatory genes in certain patient populations (e.g., co-stimulatory genes and/or pathways described in Lincecum et al. in Nature Genetics, Advanced Online Publication March 28, 2010).

In some of the methods described herein, following administration of ACTH to an individual diagnosed with, suspected of having, or predisposed to ALS, the therapeutic efficacy of a dosing regimen of the ACTH preparation, ACTH peptide or fragment, analog, complex or aggregate thereof, or any combination thereof, as described herein, may be evaluated by determination of expression of upregulated genes in the co-stimulatory pathways, which serves as biomarker for disease progression and/or effect of any therapy described herein.

### Dosing Regimen

In some of the methods of treatment of ALS described above, the first dose and one or more subsequent doses of ACTH or fragment, analog, complex or aggregate thereof, or any combination thereof, may be administered in a dosing regimen that is a pulsed dosing regimen (e.g., the dosing schedule produces escalating ACTH levels early in the dosing interval followed by a prolonged dose-free period). In some of the methods of treatment of ALS described above, the first dose and one or more subsequent doses of ACTH or fragment, analog, complex or aggregate thereof, or any combination thereof, may be administered in a dosing regimen that is not continuous (i.e., the intervals between doses are uneven). In some of the methods of treatment of ALS described above, the first dose and one or more subsequent doses of ACTH or fragment, analog, complex or aggregate thereof, or any combination thereof, may be administered in a dosing regimen that is a continuous dosing regimen.

The first dose may be administered upon detection of one or more symptoms of ALS and/or a mutation in the SOD1 gene. The first dose may be administered upon detection of excess glutamate levels in the CSF and/or reduced expression of VEGF receptors on motor neurons and/or Bunina bodies in motor neurons. The one or more subsequent doses may be administered every day, every other day, every two days, every three days, every four days, every 5 days, every 6 days, once a week, every two weeks, every three weeks, once a month, every six weeks, every two months, every three months, every four months five months, every six months or any combination thereof.

The dosing regimen comprises doses that may produce decreasing levels of drug early in the dosing interval followed by a prolonged dose-free interval. The dosing regimen may comprises a first dose, a series of subsequent doses, followed by a drug holiday, and then, one or more series of doses that are the same as or different from the first series of doses. By way of example only, in one dosing regimen, the methods of treatment of ALS describe above comprise administration of ACTH or fragment, analog, complex or aggregate thereof, or any combination thereof, and comprise a first dose of 80 IU, then a once daily dose of 20 IU for three days, followed by a 40 IU dose every week for a month, followed by a drug holiday for 3 months, and then a second series of doses comprising a first dose of 60 IU, then a once daily dose of 20 IU for three days, followed by a 40 IU dose every week for a month, followed by a drug holiday for 3 months.

A dosing regimen may comprise dosing that produces escalating levels of drug early in the dosing interval followed by a prolonged dose-free period. By way of example only, in one dosing regimen, the methods of treatment of ALS describe above comprise administration of ACTH or fragment, analog, complex or aggregate thereof, or any combination thereof, and comprise a first dose of 20 IU, a second dose of 20 IU in the same week, then 40 IU twice a week, then 40 IU every other month for three months.

A first dose of ACTH or fragment, analog, complex or aggregate thereof, or any combination thereof, may be between about 10 IU, 20 IU, 30 IU, 40 IU, 50 IU, 60 IU, 70 IU, 80 IU to about 50 IU, 60 IU, 70 IU, 80 IU, 90 IU, 100 IU, 110 IU, 120 IU, 130 IU, 140 IU, 150 IU or 200 IU. A first dose of ACTH or fragment, analog, complex or aggregate thereof, or any combination thereof, may be between about 10 IU to about 200 IU, between about 10 IU to about 150 IU, between about 10 IU to about 100 IU, between about 10 IU to about 80 IU, between about 10 IU to about 60 IU, or between about 10 IU to about 40 IU. A first dose of ACTH or fragment, analog, complex or aggregate thereof, or any combination thereof, may be between about 10 IU to about 200 IU, between about 20 IU to about 200 IU, between about 40 IU to about 200 IU, between about 40 IU to about 150 IU, between about 40 IU to about 100 IU, between about 40 IU to about 80 IU, or between about 40 IU to about 60 IU. A first dose of ACTH or fragment, analog, complex or aggregate thereof, or any combination thereof, may be between about 20 IU to about 200 IU, between about 60 IU to about 150 IU, between about 60 IU to about 100 IU, or between about 60 IU to about 80 IU.

A one or more subsequent dose of ACTH or fragment, analog, complex or aggregate thereof, or any combination thereof, may be between about 10 IU, 20 IU, 30 IU, 40 IU, 50 IU, 60 IU, 70 IU, 80 IU to about 50 IU, 60 IU, 70 IU, 80 IU, 90 IU, 100 IU, 110 IU, 120 IU, 130 IU, 140 IU, 150 IU or 200 IU. A one or more subsequent dose of ACTH or fragment, analog, complex or aggregate thereof, or any combination thereof, may be between about 10 IU to about 200 IU, between about 10 IU to about 150 IU, between about 10 IU to about 100 IU, between about 10 IU to about 80 IU, between about 10 IU to about 60 IU, or between about 10 IU to about 40 IU. A one or more subsequent dose of ACTH or fragment, analog, complex or aggregate thereof, or any combination thereof, may be between about 20 IU to about 200 IU, between about 20 IU to about 150 IU, between about 20 IU to about 100 IU, between about 20 IU to about 80 IU, or between about 20 IU to about 60 IU, or between about 20 IU to about 40 IU. A one or more subsequent dose of ACTH or fragment, analog, complex or aggregate thereof, or any combination thereof, may be between about 40 IU to about 200 IU, between about 40 IU to about 150 IU, between about 40 IU to about 100 IU, between about 40 IU to about 80 IU, or between about 40 IU to about 60 IU. A one or more subsequent dose of ACTH or fragment, analog, complex or aggregate thereof, or any combination thereof, may be between about 20 IU to about 200 IU, between about 60 IU to about 150 IU, between about 60 IU to about 100 IU, or between about 60 IU to about 80 IU.

A first dose of ACTH or fragment, analog, complex or aggregate thereof, or any combination thereof, may be between about 10 mg, 20 mg, 30 mg, 40 mg, 50 mg, 60 mg, 70 mg, 80 mg to about 50 mg, 60 mg, 70 mg, 80 mg, 90 mg, 100 mg, 110 mg, 120 mg, 130 mg, 140 mg, 150 mg or 200 mg. In some embodiments, a first dose of ACTH or fragment, analog, complex or aggregate thereof, or any combination thereof, may be between about 10 mg to about 200 mg, between about 20 mg to about 200 mg, between about 20 mg to about 150 mg, between about 20 mg to about 100 mg, between about 20 mg to about 80 mg, between about 20 mg to about 60 mg, or between about 20 mg to about 40 mg. A first dose of ACTH or fragment, analog, complex or aggregate thereof, or any combination thereof, may be between about 40 mg to about 200 mg, between about 40 mg to about 150 mg, between about 40 mg to about 100 mg, between about 40 mg to about 80 mg, between about 50 mg to about 70 mg or between about 40 mg to about 60 mg. A first dose of ACTH or fragment, analog, complex or aggregate thereof, or any combination thereof, may be between about 20 mg to about 200 mg, between about 60 mg to about 150 mg, between about 60 mg to about 100 mg, or between about 60 mg to about 80 mg.

A one or more subsequent dose of ACTH or fragment, analog, complex or aggregate thereof, or any combination thereof, may be between about 10 mg, 20 mg, 30 mg, 40 mg, 50 mg, 60 mg, 70 mg, 80 mg to about 50 mg, 60 mg, 70 mg, 80 mg, 90 mg, 100 mg, 110 mg, 120 mg, 130 mg, 140 mg, 150 mg or 200 mg. A one or more subsequent dose of ACTH or fragment, analog, complex or aggregate thereof, or any combination thereof, may be between about 10 mg to about 200 mg, between about 20 mg to about 200 mg, between about 20 mg to about 150 mg, between about 20 mg to about 100 mg, between about 20 mg to about 80 mg, between about 20 mg to about 60 mg, or between about 20 mg to about 40 mg. A one or more subsequent dose of ACTH or fragment, analog, complex or aggregate thereof, or any combination thereof, may be between about 40 mg to about 200 mg, between about 40 mg to about 150 mg, between about 40 mg to about 100 mg, between about 40 mg to about 80 mg, between about 50 mg to about 70 mg or between about 40 mg to about 60 mg. A one or more subsequent dose of ACTH or fragment, analog, complex or aggregate thereof, or any combination thereof, may be between about 20 mg to about 200 mg, between about 60 mg to about 150 mg, between about 60 mg to about 100 mg, or between about 60 mg to about 80 mg.

A first dose of ACTH or fragment, analog, complex or aggregate thereof, or any combination thereof, may be between about 10 U/kg to about 200 U/kg, between about 20 mg/kg to about 200 U/kg, between about 20 U/kg to about 150 U/kg, between about 20 U/kg to about 100 U/kg, between about 20 U/kg to about 80 U/kg, between about 20 U/kg to about 60 U/kg, or between about 20 U/kg to about 40 U/kg. A first dose of ACTH or fragment, analog, complex or aggregate thereof, or any combination thereof, may be between about 40 U/kg to about 200 U/kg, between about 40 U/kg to about 150 U/kg, between about 40 U/kg to about 100 U/kg, between about 40 U/kg to about 80 U/kg, between about 50 U/kg to about 70 U/kg, or between about 40 U/kg to about 60 U/kg. A first dose of ACTH or fragment, analog, complex or aggregate thereof, or any combination thereof, may be between about 20 U/kg to about 200 U/kg, between about 60 U/kg to about 150 U/kg, between about 60 U/kg to about 100 U/kg, or between about 60 U/kg to about 80 U/kg.

A one or more subsequent dose of ACTH or fragment, analog, complex or aggregate thereof, or any combination thereof, may be between about 10 U/kg to about 200 U/kg, between about 20 mg/kg to about 200 U/kg, between about 20 U/kg to about 150 U/kg, between about 20 U/kg to about 100 U/kg, between about 20 U/kg to about 80 U/kg, between about 20 U/kg to about 60 U/kg, or between about 20 U/kg to about 40 U/kg. A one or more subsequent dose of ACTH or fragment, analog, complex or aggregate thereof, or any combination thereof, may be between about 40 U/kg to about 200 U/kg, between about 40 U/kg to about 150 U/kg, between about 40 U/kg to about 100 U/kg, between about 40 U/kg to about 80 U/kg, between about 50 U/kg to about 70 U/kg, or between about 40 U/kg to about 60 U/kg. A one or more subsequent dose of ACTH or fragment, analog, complex or aggregate thereof, or any combination thereof, may be between about 20 U/kg to about 200 U/kg, between about 60 U/kg to about 150 U/kg, between about 60 U/kg to about 100 U/kg, or between about 60 U/kg to about 80 U/kg.

Where the ACTH, or fragment, analog, complex or aggregate thereof, or any combination thereof, is a synthetic preparation (i.e., not naturally occurring), a first dose of ACTH or fragment, analog, complex or aggregate thereof, or any combination thereof, may be between about 10 mg/kg, 20 mg/kg, 30 mg/kg, 40 mg/kg, 50 mg/kg, 60 mg/kg, 70 mg/kg, 80 mg/kg to about 50 mg/kg, 60 mg/kg, 70 mg/kg, 80 mg/kg, 90 mg/kg, 100 mg/kg, 110 mg/kg, 120 mg/kg, 130 mg/kg, 140 mg/kg, 150 mg/kg or 200 mg/kg. A first dose of ACTH or fragment, analog, complex or aggregate thereof, or any combination thereof, may be between about 10 mg/kg to about 200 mg/kg, between about 20 mg/kg to about 200 mg/kg, between about 20 mg/kg to about 150 mg/kg, between about 20 mg/kg to about 100 mg/kg, between about 20 mg/kg to about 80 mg/kg, between about 20 mg/kg to about 60 mg/kg, or between about 20 mg/kg to about 40 mg/kg. A first dose of ACTH or fragment, analog, complex or aggregate thereof, or any combination thereof, may be between about 40 mg/kg to about 200 mg/kg, between about 40 mg/kg to about 150 mg/kg, between about 40 mg/kg to about 100 mg/kg, between about 40 mg/kg to about 80 mg/kg, between about 50 mg/kg to about 70 mg/kg or between about 40 mg/kg to about 60 mg/kg. A first dose of ACTH or fragment, analog, complex or aggregate thereof, or any combination thereof, may be between about 20 mg/kg to about 200 mg/kg, between about 60 mg/kg to about 150 mg/kg, between about 60 mg/kg to about 100 mg/kg, or between about 60 mg/kg to about 80 mg/kg.

Where the ACTH, or fragment, analog, complex or aggregate thereof, or any combination thereof, is a synthetic preparation (i.e., not naturally occurring), a one or more subsequent dose of ACTH or fragment, analog, complex or aggregate thereof, or any combination thereof, may be between about 10 mg/kg, 20 mg/kg, 30 mg/kg, 40 mg/kg, 50 mg/kg, 60 mg/kg, 70 mg/kg, 80 mg/kg to about 50 mg/kg, 60 mg/kg, 70 mg/kg, 80 mg/kg, 90 mg/kg, 100 mg/kg, 110 mg/kg, 120 mg/kg, 130 mg/kg, 140 mg/kg, 150 mg/kg or 200 mg/kg. A one or more subsequent dose of ACTH or fragment, analog, complex or aggregate thereof, or any combination thereof, may be between about 10 mg/kg to about 200 mg/kg, between about 10 mg/kg to about 150 mg/kg, between about 10 mg/kg to about 100 mg/kg, between about 10 mg/kg to about 80 mg/kg, between about 10 mg/kg to about 60 mg/kg, or between about 10 mg/kg to about 40 mg/kg. A one or more subsequent dose of ACTH or fragment, analog, complex or aggregate thereof, or any combination thereof, may be between about 20 mg/kg to about 200 mg/kg, between about 20 mg/kg to about 150 mg/kg, between about 20 mg/kg to about 100 mg/kg, between about 20 mg/kg to about 80 mg/kg, or between about 20 mg/kg to about 60 mg/kg. A one or more subsequent dose of ACTH or fragment, analog, complex or aggregate thereof, or any combination thereof, may be between about 40 mg/kg to about 200 mg/kg, between about 40 mg/kg to about 150 mg/kg, between about 40 mg/kg to about 100 mg/kg, between about 40 mg/kg to about 80 mg/kg, or between about 40 mg/kg to about 60 mg/kg. A one or more subsequent dose of ACTH or fragment, analog, complex or aggregate thereof, or any combination thereof, may be between about 20 mg/kg to about 200 mg/kg, between about 60 mg/kg to about 150 mg/kg, between about 60 mg/kg to about 100 mg/kg, or between about 60 mg/kg to about 80 mg/kg.

A one or more subsequent dose of ACTH or fragment, analog, complex or aggregate thereof, or any combination thereof, may be between about 10% - 90%, between about 20% - 80%, between about 20% - 60%, or between about 20% - 40% of the first dose of ACTH or fragment, analog, complex or aggregate thereof, or any combination thereof. A one or more subsequent dose of ACTH or fragment, analog, complex or aggregate thereof, or any combination thereof, may be between about 80% - 200%, between about 80% - 175%, between about 80% - 150%, between about 80% - 125%, or between about 80% - 100% of the first dose of ACTH or fragment, analog, complex or aggregate thereof, or any combination thereof.

The ACTH peptide or fragment, analog, complex or aggregate thereof, or any combination thereof, may be administered to an individual in need thereof in an amount sufficient to provide plasma cortisol secretion levels between about 1.1 to about 10 times 1.1 to about 8 times, 1.1 to about 6 times, 1.1 to about 4 times, between about 1.1 to about 3 times, between about 1.1 to about 2 times, between about 1.1 to about 1.5 times the plasma cortisol secretion levels of a normal individual at about 8 am. The ACTH peptide or fragment, analog, complex or aggregate thereof, or any combination thereof, may be administered to an individual in need thereof in an amount sufficient to provide plasma cortisol secretion levels between about 1.5 to about 4 times, between about 1.5 to about 3 times, or between about 1.15 to about 2 times, the plasma cortisol secretion levels of a normal individual at about 8 am.

The ACTH peptide or fragment, analog, complex or aggregate thereof, or any combination thereof, may be administered to an individual in need thereof in an amount sufficient to provide plasma cortisol secretion levels between about 1.1 to about 10 times 1.1 to about 8 times, 1.1 to about 6 times, 1.1 to about 4 times, between about 1.1 to about 3 times, between about 1.1 to about 2 times, or between about 1.1 to about 1.5 times the plasma cortisol secretion levels prior to administration of the ACTH peptide or fragment, analog, complex or aggregate thereof, or any combination thereof. The ACTH peptide or fragment, analog, complex or aggregate thereof, or any combination thereof, may be administered to an individual in need thereof in an amount sufficient to provide plasma cortisol secretion levels between about 1.5 to about 4 times, between about 1.5 to about 3 times, or between about 1.15 to about 2 times, the plasma cortisol secretion levels prior to administration of the ACTH peptide or fragment, analog, complex or aggregate thereof, or any combination thereof.

The ACTH peptide or fragment, analog, complex or aggregate thereof, or any combination thereof, may be administered in an amount sufficient to provide plasma cortisol concentration between about 1.5 to about 120 µg/100 mL over at least 24 hours after administration. The ACTH peptide or fragment, analog, complex or aggregate thereof, or any combination thereof, may be administered in an amount sufficient to provide plasma cortisol concentration between about 1.5 to about 60 µg/100 mL over at least 24 hours after administration. The ACTH peptide or fragment, analog, complex or aggregate thereof, or any combination thereof, may be administered in an amount sufficient to provide plasma cortisol concentration between about 1.5 to about 30 µg/100 mL over at least 24 hours after administration.

Where the patient's condition does not improve upon administration of a dosing regimen described herein, upon the doctor's discretion the ACTH peptide or fragment, analog, complex or aggregate thereof, or any combination thereof is optionally administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the ACTH peptide or fragment, analog, complex or aggregate thereof, or any combination thereof is optionally given continuously; alternatively, the dose of ACTH peptide or fragment, analog, complex or aggregate thereof, or any combination thereof being administered is temporarily reduced or temporarily suspended for a certain length of time (i.e., a "drug holiday"). The length of the drug holiday optionally varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday includes from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's conditions has occurred, a maintenance dose is administered if necessary. Subsequently, the dosage or the frequency of administration, or both, is reduced, as a function of the symptoms, to a level at which the improved disease, disorder or condition is retained. Patients may require intermittent treatment on a long-term basis upon any recurrence of symptoms.

In some embodiments, the pharmaceutical compositions described herein are in unit dosage forms suitable for single administration of precise dosages. In unit dosage form, the formulation is divided into unit doses containing appropriate quantities of ACTH peptide or fragment, analog, complex or aggregate thereof, or any combination thereof. In some embodiments, the unit dosage is in the form of a package containing discrete quantities of the formulation. Non-limiting examples are packaged tablets or capsules, powders in vials or ampoules, or injectable suspension or solution in ampoules. In some embodiments, aqueous suspension compositions are packaged in single-dose non-reclosable containers. Alternatively, multiple-dose reclosable containers are used. In some of such embodiments, a preservative is optionally included in the composition. By way of example only, formulations for intramuscular injection are presented in unit dosage form, which include, but are not limited to ampoules, or in multi dose containers, with an added preservative.

Toxicity and therapeutic efficacy of such therapeutic regimens are optionally determined in cell cultures or experimental animals, including, but not limited to, the determination of the LD50 (the dose lethal to 50% of the population) and the ED50 (the dose therapeutically effective in 50% of the population). The dose ratio between the toxic and therapeutic effects is the therapeutic index, which is expressed as the ratio between LD50 and ED50. ACTH peptide or fragment, analog, complex or aggregate thereof, or any combination thereof, exhibiting high therapeutic indices are preferred. The data obtained from cell culture assays and animal studies (e.g., studies in G93A SOD1 mice as an animal model for ALS) is optionally used in formulating a range of dosage for use in human. The dosage of such ACTH peptide or fragment, analog, complex or aggregate thereof, or any combination thereof lies preferably within a range of circulating concentrations that include the ED50 with minimal toxicity. The dosage optionally varies within this range depending upon the dosage form employed and the route of administration utilized.

### Combination therapy

In some of the methods and dosing regimens described above, ACTH peptide or fragment, analog, complex or aggregate thereof, or any combination thereof, is administered in combination with other agents including, and not limited to, riluzole, ceftriaxone, methylcobalamine, Aeolus 10150, edaravone, hepatocyte growth factor (HGF), insulin growth factor (IGF), Atorvastatin, Lithium carbonate, Avanier 07-ACR-123 (Zenvia®), SB-509, Talampanel, Thalidomide, Arimoclomol, Olanzapine, KNS-760704, memantine, tamoxifen, ONO-2506PO, MCI-186, pioglitazone, ALS-357, creatine monohydrate, TCH346, Botulinum toxin type B, tauroursodeoxycholic acid, Dronabinol, coenzyme Q10, YAM80, Olesoxime, escitalopram (Lexapro®), sodium phenylbutyrate, ISIS 333611, granulocyte stimulating factor, neuronal growth factor (NGF), brain-derived neurotrophic factor (BDNF), neurotrophin 3 (NT3), basic fibroblast growth factor (bFGF), R(+) pramipexole dihydrochloride monohydrate, Sodium Valproate, AVP-923, sNN0029, Antithymocyte globulin, cyclosporin, corticosteroids, modafinil. A second therapeutic agent may be an antibody or antibody fragment (e.g., CD40L monoclonal antibody).

In some of the methods and dosing regimens described above, ACTH peptide or fragment, analog, complex or aggregate thereof, or any combination thereof, is administered in combination with agents that are used to treat symptoms of ALS such as fatigue, excessive salivation, pain, depression, excessive phlegm or constipation. In some of the methods and dosing regimens described above, ACTH peptide or fragment, analog, complex or aggregate thereof, or any combination thereof, is administered in combination with devices such as intramuscular diaphragm electrodes or diaphragmatic pacer implants. In some of the methods and dosing regimens described above, ACTH peptide or fragment, analog, complex or aggregate thereof, or any combination thereof, is administered in combination with siRNA (e.g., siRNA specific for the SOD1 mutated gene) or an antibody. siRNA may be delivered using any suitable method including and not limited to vector delivery methods. In some of the methods and dosing regimens described above, ACTH peptide or fragment, analog, complex or aggregate thereof, or any combination thereof, is administered in combination with stem cell transplant (e.g., intraspinal infusion of autologous bone marrow stem cells).

In combination therapy, the ACTH peptide or fragment, analog, complex or aggregate thereof, or any combination thereof, and the second therapeutic agent may be administered simultaneously. In combination therapy, the ACTH peptide or fragment, analog, complex or aggregate thereof, or any combination thereof, and the second therapeutic agent may be administered serially in any order. In combination therapy, the ACTH peptide or fragment, analog, complex or aggregate thereof, or any combination thereof, and the second therapeutic agent may be administered at different intervals. By way of example only, a second therapeutic agent is administered after completion of a dosing regimen comprising administration of ACTH peptide or fragment, analog, complex or aggregate thereof, or any combination thereof.

### Pharmaceutical formulations

Provided herein, in certain embodiments, are compositions comprising at least one ACTH peptide, where the ACTH peptide is as described herein.

Pharmaceutical compositions are formulated using one or more physiologically acceptable carriers including excipients and auxiliaries which facilitate processing of the ACTH peptide into preparations which are used pharmaceutically. Proper formulation is dependent upon the route of administration chosen. A summary of pharmaceutical compositions is found, for example, in Remington: The Science and Practice of Pharmacy, Nineteenth Ed (Ea hston, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins, 1999).

Provided herein are pharmaceutical compositions that include ACTH peptide, and a pharmaceutically acceptable diluent(s), excipient(s), or carrier(s). In addition, the ACTH peptide is optionally administered as pharmaceutical compositions in which it is mixed with other active ingredients, as in combination therapy. In some embodiments, the pharmaceutical compositions includes other medicinal or pharmaceutical agents, carriers, adjuvants, such as preserving, stabilizing, wetting or emulsifying agents, solution promoters, salts for regulating the osmotic pressure, and/or buffers. In addition, the pharmaceutical compositions also contain other therapeutically valuable substances.

A pharmaceutical composition, as used herein, refers to a mixture of ACTH peptide or fragment, analog, complex or aggregate thereof, or any combination thereof, with other chemical components, such as carriers, stabilizers, diluents, dispersing agents, suspending agents, thickening agents, and/or excipients. In some embodiments, a pharmaceutical composition comprises an ACTH preparation (e.g., an ACTH peptide or fragment, analog, complex or aggregate thereof, or any combination thereof, and any other proteins and/or other substances that are present in a homogenized pituitary extract obtained from an appropriate animal source) and other chemical components, such as carriers, stabilizers, diluents, dispersing agents, suspending agents, thickening agents, and/or excipients. The pharmaceutical composition facilitates administration of ACTH peptide to an organism. In practicing the methods of treatment or use provided herein, an ACTH peptide is administered in a pharmaceutical composition to a mammal having a condition, disease, or disorder to be treated. Preferably, the mammal is a human. The does and dosing regimen varies depending on the severity and stage of the condition, the age and relative health of an individual, the potency of ACTH peptide used and other factors. The ACTH peptide is optionally used singly or in combination with one or more therapeutic agents as components of mixtures.

The pharmaceutical formulations described herein are optionally administered to an individual by multiple administration routes, including but not limited to, oral, parenteral (e.g., intravenous, subcutaneous, intramuscular, intrathecal), intranasal, buccal, topical, rectal, or transdermal administration routes. The pharmaceutical formulations described herein include, but are not limited to, aqueous liquid dispersions, self-emulsifying dispersions, solid solutions, liposomal dispersions, aerosols, solid dosage forms, powders, immediate release formulations, controlled release formulations, fast melt formulations, tablets, capsules, pills, delayed release formulations, extended release formulations, pulsatile release formulations, multiparticulate formulations, and mixed immediate and controlled release formulations.

The pharmaceutical compositions will include at least one ACTH peptide as an active ingredient in free-acid or free-base form, or in a pharmaceutically acceptable salt form. In addition, the methods and pharmaceutical compositions described herein include the use of N-oxides, crystalline forms (also known as polymorphs), as well as active metabolites of ACTH peptide having the same type of activity. In some situations, ACTH peptide exist as tautomers and/or rotational isomers. All tautomers and/or rotational isomers are included within the scope of the embodiments presented herein. Additionally, ACTH peptide exists in unsolvated as well as solvated forms with pharmaceutically acceptable solvents such as water, ethanol. The solvated forms of the ACTH peptide presented herein are also considered to be disclosed herein. In some embodiments, ACTH peptide exists as a complex with metal ions. The metal-ion complexed forms of the ACTH peptide presented herein are also considered to be disclosed herein.

"Carrier materials" include any commonly used excipients in pharmaceutics and should be selected on the basis of compatibility with ACTH peptide disclosed herein, and the release profile properties of the desired dosage form. Exemplary carrier materials include, e.g., binders, suspending agents, disintegration agents, filling agents, surfactants, solubilizers, stabilizers, lubricants, wetting agents, diluents.

Moreover, the pharmaceutical compositions described herein, which include an ACTH peptide are formulated into any suitable dosage form, including but not limited to, aqueous oral dispersions, liquids, gels, syrups, elixirs, slurries, suspensions, for oral ingestion by a patient to be treated, solid oral dosage forms, aerosols, controlled release formulations, fast melt formulations, effervescent formulations, lyophilized formulations, tablets, powders, pills, dragees, capsules, delayed release formulations, extended release formulations, pulsatile release formulations, multiparticulate formulations, and mixed immediate release and controlled release formulations. In some embodiments, a formulation comprising an ACTH peptide is a solid drug dispersion. A solid dispersion is a dispersion of one or more active ingredients in an inert carrier or matrix at solid state prepared by the melting (or fusion), solvent, or melting-solvent methods. (Chiou and Riegelman, Journal of Pharmaceutical Sciences, 60, 1281 (1971)). The dispersion of one or more active agents in a solid diluent is achieved without mechanical mixing. Solid dispersions are also called solid-state dispersions. In some embodiments, any ACTH peptide described is formulated as a spray dried dispersion (SDD). An SDD is a single phase amorphous molecular dispersion of a drug in a polymer matrix. It is a solid solution prepared by dissolving the drug and a polymer in a solvent (e.g., acetone, methanol) and spray drying the solultion. The solvent rapidly evaporates from droplets which rapidly solidifies the polymer and drug mixture trapping the drug in amorphous form as an amorphous molecular dispersion. In some embodiments, such amorphous dispersions are filled in capsules and/or constituted into powders for reconstitution. Solubility of an SDD comprising a drug is higher than the solubility of a crystalline form of a drug or a non-SDD amorphous form of a drug. In some of the methods described herein, ACTH peptide or fragment, analog, complex or aggregate thereof, or any combination thereof, are administered as SDDs constituted into appropriate dosage forms described herein.

Pharmaceutical preparations for oral use are optionally obtained by mixing one or more solid excipient with an ACTH peptide, optionally grinding the resulting mixture, and processing the mixture of granules, after adding suitable auxiliaries, if desired, to obtain tablets or dragee cores. Suitable excipients include, for example, fillers such as sugars, including lactose, sucrose, mannitol, or sorbitol; cellulose preparations such as, for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. If desired, disintegrating agents are added, such as the cross linked croscarmellose sodium, polyvinylpyrrolidone, agar, or alginic acid or a salt thereof such as sodium alginate. A prodrug of the ACTH peptide or fragment, analog, complex or aggregate thereof, or any combination thereof, may be used in preparations for oral use.

Dragee cores are provided with suitable coatings. For this purpose, concentrated sugar solutions are generally used, which optionally contain gum arabic, talc, polyvinylpyrrolidone, carbopol gel, polyethylene glycol, and/or titanium dioxide, lacquer solutions, and suitable organic solvents or solvent mixtures. Dyestuffs or pigments are optionally added to the tablets or dragee coatings for identification or to characterize different combinations of active compound doses.

In some embodiments, the solid dosage forms disclosed herein are in the form of a tablet, (including a suspension tablet, a fast-melt tablet, a bite-disintegration tablet, a rapid-disintegration tablet, an effervescent tablet, or a caplet), a pill, a powder (including a sterile packaged powder, a dispensable powder, or an effervescent powder) a capsule (including both soft or hard capsules, e.g., capsules made from animal-derived gelatin or plant-derived HPMC, or "sprinkle capsules"), solid dispersion, solid solution, bioerodible dosage form, controlled release formulations, pulsatile release dosage forms, multiparticulate dosage forms, pellets, granules, or an aerosol. In other embodiments, the pharmaceutical formulation is in the form of a powder. In still other embodiments, the pharmaceutical formulation is in the form of a tablet, including but not limited to, a fast-melt tablet. Additionally, pharmaceutical formulations of an ACTH peptide or fragment, analog, complex or aggregate thereof, or any combination thereof, are optionally administered as a single capsule or in multiple capsule dosage form. In some embodiments, the pharmaceutical formulation is administered in two, or three, or four, capsules or tablets.

Dosage forms may include microencapsulated formulations. One or more other compatible materials may be present in the microencapsulation material. Exemplary materials include, but are not limited to, pH modifiers, erosion facilitators, anti-foaming agents, antioxidants, flavoring agents, and carrier materials such as binders, suspending agents, disintegration agents, filling agents, surfactants, solubilizers, stabilizers, lubricants, wetting agents, and diluents.

Exemplary microencapsulation materials useful for delaying the release of the formulations including an ACTH peptide or fragment, analog, complex or aggregate thereof, or any combination thereof, include, but are not limited to, hydroxypropyl cellulose ethers (HPC) such as Klucel® or Nisso HPC, low-substituted hydroxypropyl cellulose ethers (L-HPC), hydroxypropyl methyl cellulose ethers (HPMC) such as Seppifilm-LC, Pharmacoat®, Metolose SR, Methocel®-E, Opadry YS, PrimaFlo, Benecel MP824, and Benecel MP843, methylcellulose polymers such as Methocel®-A, hydroxypropylmethylcellulose acetate stearate Aqoat (HF-LS, HF-LG,HF-MS) and Metolose®, Ethylcelluloses (EC) and mixtures thereof such as E461, Ethocel®, Aqualon®-EC, Surelease®, Polyvinyl alcohol (PVA) such as Opadry AMB, hydroxyethylcelluloses such as Natrosol®, carboxymethylcelluloses and salts of carboxymethylcelluloses (CMC) such as Aqualon®-CMC, polyvinyl alcohol and polyethylene glycol co-polymers such as Kollicoat IR®, monoglycerides (Myverol), triglycerides (KLX), polyethylene glycols, modified food starch, acrylic polymers and mixtures of acrylic polymers with cellulose ethers such as Eudragit® EPO, Eudragit® L30D-55, Eudragit® FS 30D Eudragit® L100-55, Eudragit® L100, Eudragit® S100, Eudragit® RD100, Eudragit® E100, Eudragit® L12.5, Eudragit® S12.5, Eudragit® NE30D, and Eudragit® NE 40D, cellulose acetate phthalate, sepifilms such as mixtures of HPMC and stearic acid, cyclodextrins, and mixtures of these materials.

The pharmaceutical solid oral dosage forms including formulations described herein, which include an ACTH peptide, are optionally further formulated to provide a controlled release (also known as modified release) of the ACTH peptide. As used herein, a modified release or controlled release refers to the release of the ACTH peptide from a dosage form in which it is incorporated according to a desired profile over an extended period of time. Such modified and/or controlled release profiles include, for example, sustained release, prolonged release, pulsatile release, and delayed release profiles. In contrast to immediate release compositions, controlled release or modified release compositions allow delivery of an agent to an individual over an extended period of time according to a predetermined profile. Such release rates provide levels of ACTH peptide for an extended period of time and thereby provide a longer period of pharmacologic response while minimizing side effects as compared to conventional rapid release dosage forms. Such longer periods of response provide for many inherent benefits that are not achieved with the corresponding short acting, immediate release preparations.

In other embodiments, the formulations described herein, which include an ACTH peptide, are delivered using a pulsatile dosage form. A pulsatile dosage form is capable of providing one or more immediate release pulses at predetermined time points after a controlled lag time or at specific sites. Pulsatile dosage forms including the formulations described herein, which include an ACTH peptide, are optionally administered using a variety of pulsatile formulations that include, but are not limited to, those described in U.S. Pat. Nos. 5,011,692, 5,017,381, 5,229,135, and 5,840,329. Other pulsatile release dosage forms suitable for use with the present formulations include, but are not limited to, for example, U.S. Pat. Nos. 4,871,549, 5,260,068, 5,260,069, 5,508,040, 5,567,441 and 5,837,284.

In further embodiments, the formulations described herein, which include an ACTH peptide, are delivered as immediate release formulations (e.g., intravenously).

Additional embodiments include oral administration of an ACTH peptide. Oral administration is optionally in the form of a solid dosage form (e.g., a gelatin capsule), or liquid dosage form. Liquid formulation dosage forms for oral administration are optionally aqueous suspensions selected from the group including, but not limited to, pharmaceutically acceptable aqueous oral dispersions, emulsions, solutions, elixirs, gels, and syrups. See, e.g., Singh et al., Encyclopedia of Pharmaceutical Technology, 2nd Ed., pp. 754-757 (2002). In addition to the ACTH, the liquid dosage forms optionally include additives, such as: (a) disintegrating agents; (b) dispersing agents; (c) wetting agents; (d) at least one preservative, (e) viscosity enhancing agents, (f) at least one sweetening agent, and (g) at least one flavoring agent. In some embodiments, the aqueous dispersions further includes a crystal-forming inhibitor.

In some embodiments, the pharmaceutical formulations described herein are self-emulsifying drug delivery systems (SEDDS). Emulsions are dispersions of one immiscible phase in another, usually in the form of droplets. Generally, emulsions are created by vigorous mechanical dispersion. SEDDS, as opposed to emulsions or microemulsions, spontaneously form emulsions when added to an excess of water without any external mechanical dispersion or agitation. An advantage of SEDDS is that only gentle mixing is required to distribute the droplets throughout the solution. Additionally, water or the aqueous phase is optionally added just prior to administration, which ensures stability of an unstable or hydrophobic active ingredient. Thus, the SEDDS provides an effective delivery system for oral and parenteral delivery of hydrophobic active ingredients. In some embodiments, SEDDS provides improvements in the bioavailability of hydrophobic active ingredients. Methods of producing self-emulsifying dosage forms include, but are not limited to, for example, U.S. Pat. Nos. 5,858,401, 6,667,048, and 6,960,563.

Suitable intranasal formulations include those described in, for example, U.S. Pat. Nos. 4,476,116, 5,116,817 and 6,391,452. Nasal dosage forms generally contain large amounts of water in addition to the active ingredient. Minor amounts of other ingredients such as pH adjusters, emulsifiers or dispersing agents, preservatives, surfactants, gelling agents, or buffering and other stabilizing and solubilizing agents are optionally present.

For administration by inhalation, the ACTH is optionally in a form as an aerosol, a mist or a powder. Pharmaceutical compositions described herein are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol, the dosage unit is determined by providing a valve to deliver a metered amount. Capsules and cartridges of, such as, by way of example only, gelatin for use in an inhaler or insufflator are formulated containing a powder mix of the ACTH peptide or fragment, analog, complex or aggregate thereof, or any combination thereof, and a suitable powder base such as lactose or starch.

Buccal formulations that include an ACTH peptide include, but are not limited to, U.S. Pat. Nos. 4,229,447, 4,596,795, 4,755,386, and 5,739,136. In addition, the buccal dosage forms described herein optionally further include a bioerodible (hydrolysable) polymeric carrier that also serves to adhere the dosage form to the buccal mucosa. The buccal dosage form is fabricated so as to erode gradually over a predetermined time period, wherein the delivery of the ACTH peptide is provided essentially throughout. Buccal drug delivery avoids the disadvantages encountered with oral drug administration, e.g., slow absorption, degradation of the active agent by fluids present in the gastrointestinal tract and/or first-pass inactivation in the liver. The bioerodible (hydrolysable) polymeric carrier generally comprises hydrophilic (water-soluble and water-swellable) polymers that adhere to the wet surface of the buccal mucosa. Examples of polymeric carriers useful herein include acrylic acid polymers and co, e.g., those known as "carbomers" (Carbopol®, which may be obtained from B.F. Goodrich, is one such polymer). Other components also be incorporated into the buccal dosage forms described herein include, but are not limited to, disintegrants, diluents, binders, lubricants, flavoring, colorants, preservatives. For buccal or sublingual administration, the compositions optionally take the form of tablets, lozenges, or gels formulated in a conventional manner.

Transdermal formulations of an ACTH peptide are administered for example by those described in U.S. Pat. Nos. 3,598,122, 3,598,123, 3,710,795, 3,731,683, 3,742,951, 3,814,097, 3,921,636, 3,972,995, 3,993,072, 3,993,073, 3,996,934, 4,031,894, 4,060,084, 4,069,307, 4,077,407, 4,201,211, 4,230,105, 4,292,299, 4,292,303, 5,336,168, 5,665,378, 5,837,280, 5,869,090, 6,923,983, 6,929,801 and 6,946,144.

The transdermal formulations described herein include at least three components: (1) a formulation of an ACTH peptide; (2) a penetration enhancer; and (3) an aqueous adjuvant. In addition, transdermal formulations include components such as, but not limited to, gelling agents, creams and ointment bases. In some embodiments, the transdermal formulation further includes a woven or non-woven backing material to enhance absorption and prevent the removal of the transdermal formulation from the skin. In other embodiments, the transdermal formulations described herein maintain a saturated or supersaturated state to promote diffusion into the skin.

In some embodiments, formulations suitable for transdermal administration of an ACTH peptide employ transdermal delivery devices and transdermal delivery patches and are lipophilic emulsions or buffered, aqueous solutions, dissolved and/or dispersed in a polymer or an adhesive. Such patches are optionally constructed for continuous, pulsatile, or on demand delivery of pharmaceutical agents. Still further, transdermal delivery of the ACTH peptide is optionally accomplished by means of, for example, iontophoretic patches. Additionally, transdermal patches provide controlled delivery of the ACTH peptide. The rate of absorption is optionally slowed by using rate-controlling membranes or by trapping the ACTH peptide within a polymer matrix or gel. Conversely, absorption enhancers are used to increase absorption. An absorption enhancer or carrier includes absorbable pharmaceutically acceptable solvents to assist passage through the skin. For example, transdermal devices are in the form of a bandage comprising a backing member, a reservoir containing the ACTH peptide, optionally with carriers, optionally a rate controlling barrier to deliver the ACTH peptide to the skin of the host at a controlled and predetermined rate over a prolonged period of time, and means to secure the device to the skin.

Formulations that include an ACTH suitable for intramuscular, intrathecal, subcutaneous, or intravenous injection include physiologically acceptable sterile aqueous or non-aqueous solutions, dispersions, suspensions or emulsions, and sterile powders for reconstitution into sterile injectable solutions or dispersions. Examples of suitable aqueous and non-aqueous carriers, diluents, solvents, or vehicles including water, ethanol, polyols (propyleneglycol, polyethylene-glycol, glycerol and cremophor), suitable mixtures thereof, vegetable oils (such as olive oil) and injectable organic esters such as ethyl oleate. Proper fluidity is maintained, for example, by the use of a coating such as lecithin, by the maintenance of the required particle size in the case of dispersions, and by the use of surfactants. Formulations suitable for subcutaneous injection also contain optional additives such as preserving, wetting, emulsifying, and dispensing agents.

For intravenous injections, an ACTH peptide is optionally formulated in aqueous solutions, preferably in physiologically compatible buffers such as Hank's solution, Ringer's solution, or physiological saline buffer. For transmucosal administration, penetrants appropriate to the barrier to be permeated are used in the formulation. For other parenteral injections including intrathecal and intramuscular injections, appropriate formulations include aqueous or nonaqueous solutions, preferably with physiologically compatible buffers or excipients.

Parenteral injections optionally involve bolus injection or continuous infusion. Formulations for injection are optionally presented in unit dosage form, e.g., in ampoules or in multi dose containers, with an added preservative. In some embodiments, the pharmaceutical composition described herein are in a form suitable for parenteral injection as a sterile suspensions, solutions or emulsions in oily or aqueous vehicles, and contain formulatory agents such as suspending, stabilizing and/or dispersing agents. Pharmaceutical formulations for parenteral administration include aqueous solutions of the ACTH peptide in water soluble form. Additionally, suspensions of the ACTH peptide are optionally prepared as appropriate oily injection suspensions.

In some embodiments, the ACTH peptide is administered topically and formulated into a variety of topically administrable compositions, such as solutions, suspensions, lotions, gels, pastes, medicated sticks, balms, creams or ointments. Such pharmaceutical compositions optionally contain solubilizers, stabilizers, tonicity enhancing agents, buffers and preservatives.

The ACTH peptide is also optionally formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone and PEG. In suppository forms of the compositions, a low-melting wax such as, but not limited to, a mixture of fatty acid glycerides, optionally in combination with cocoa butter is first melted.

### EXAMPLES

### Example 1: In vivo testing in rats for improvement of Motor Nerve Regeneration

Male Sprague Dawley rats weighing 125-150 g are maintained on a 12 hr light: 12 hr dark cycle and supplied with rat chow and water ad lib. The animals are divided into 3 groups: (1) intact; (2) denervated controls which receive 0.2 ml saline IP 3 hr after crush denervation and thereafter every 48 hr until one day prior to the electromechanical recordings; (3) 3 h after crush denervation, treated with a first dose of 20 IU ACTHAR® gel, a subsequent dose of 20 IU ACTHAR® gel in the same week, 40 IU ACTHAR® gel twice a week and then every other month for three months.

Denervation procedure is carried out as described by Strand et al. in Peptides, 1988, 9, 215-221. Mechanical recordings and Motor Unit Performance are determined as described by Strand et al.

Significance of differences among groups is determined by means of analysis of variance. Significance between specific means is tested by the Student Newman-Keuls test. An improvement in Tetanic tension of EDL muscle is indicative of beneficial effect of a pulsed dosing regimen of ACTHAR® gel.

### Example 2: Animal model of ALS for testing neurological deficits and survival

Transgenic mice carrying high copy numbers of the transgene with the G93A human SOD1 mutation are used in this study which is a modification of the study described by Feng et al., Neuroscience, 2008, 155, 567-572. All transgenic mice are genotyped by PCR amplification of DNA extracted from the tails to identify the SOD1 mutation.

Mice are divided into vehicle and treatment groups. ACTHAR® gel treatment is initiated 30 days after birth and continued until the end stage. Each animal is given a first dose followed by a subsequent weekly dose of ACTHAR® gel. All animals are maintained on a 12 hours light/dark cycle. Behavior tests are performed during the light period. Various tests are routinely performed starting from 12 weeks of age until death.

Rotarod performance test: Motor coordination is assessed by measuring the length of time for which mice remained on the rotating rod (16 r.p.m.). Three trials are given to each animal and the longest retention time is used as a measure of competence at the task. The evaluation scores are: grade 0, >180 s; grade 1, 60-180 s; grade 2, <60 s; grade 3, falling off the rod before rotation.

Postural reflex test: This is conducted essentially as described by Bederson et al., Stroke, 1986, 17, 472-476 to examine the strength of the forelimbs. The deficits are scored as follows: grade 0, no evidence of paralysis; grade 1, forelimb flexion upon tail suspension; grade 2, decreased resistance to lateral push (and forelimb flexion) without circling; grade 3, same as grade 2 but with circling; grade 4, unable to walk but maintaining upright body position; grade 5, complete paralysis.

Screen test: This test serves as an indicator of general muscle strength. The animal is placed on a horizontally positioned screen with grids. The screen is then rotated to the vertical position. The deficit scores are: grade 0, grasping the screen with forepaws for more than 5 s; grade 1, temporarily holding the screen without falling off; grade 2, same as grade 1 but falling off within 5 s; grade 3, falling off instantaneously.

An improvement or stabilization in any of the above scores is indicative of a therapeutic effect of ACTHAR® gel.

### Example 3 : Treatment of ALS by administration of ACTHAR® gel injections

G93A SOD1 (G1H, high copy) transgenic mice - 16 control animals and 56 treated animals - were included in this study. Control animals were injected with 5% gelatin. Test animals were divided into groups of 5-9 animals where each group was injected intramuscularly or subcutaneously with ACTHAR® gel as follows and as shown below in Table 1: IM 120 U/kg 2day interval (i.e., every other day); SC 120 U/kg 2 day interval (i.e., every other day); SC 60U/kg 2 day interval (i.e., every other day); SC 60 U/kg 7 day interval.

**Table 1**

| Males | | | | | Females | | | | |
|---|---|---|---|---|---|---|---|---|---|
| 5% Gelatin | 120 IM | 120 SC | 60 SC | 60 SC, weekly | 5% Gelatin | 120 IM | 120 SC | 60 SC | 60 SC, weekly |
| 8 | 7 | 9 | 6 | 5 | 8 | 7 | 9 | 8 | 5 |

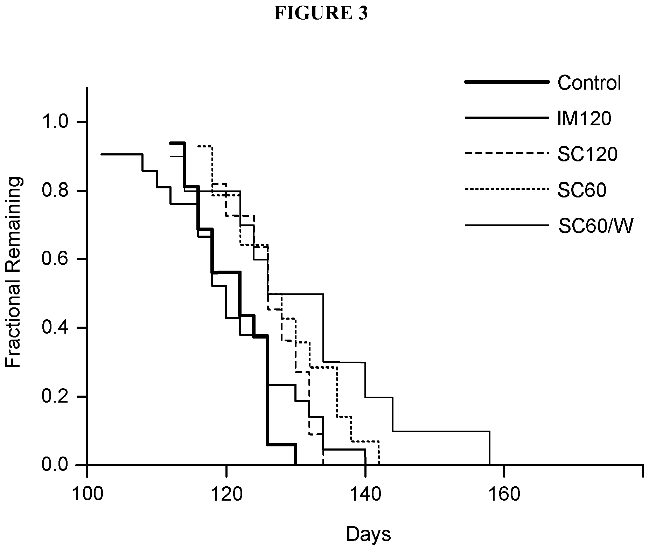

All animals were maintained on a 12 hours light/dark cycle. Behavior tests were performed during the light period. Various tests for tremor and paralysis were routinely performed starting from injections of ACTHAR® gel until death. Figure 2 and Figure 3 show the results of these tests.

Table 2, Figure 2, Figure 3 and Figure 4 show that animals treated with ACTHAR® gel show a delay in onset of tremor, and a trend for reduced paralysis and increased survival. Figure 5 shows decreased expression and deposition of SOD1 protein in treated animals in various brain and spinal cord tissues. Figure 6 shows staining of anterior horn of the lumbar segment of the spinal cord illustrating decreased expression and deposition of SOD1 protein in treated animals.

**Table 2**

| **Clinical Statistics** | | | |
|---|---|---|---|
| **Animal Group** | **Onset/Tremor Median Age** | **Paralysis Median Age** | **Endstage Median Age** |
| Control (16) | 103 | 122 | 129 |
| IM 120 (14) | 109 | 120 | 127 |
| SC 120(18) | 116 | 126 | 130 |
| SC 60 (14) | 121 | 127 | 134 |
| SC 60/W (10) | 115 | 130 | 138 |
| Log-rank significance p= | 0.0001 | 0.0509 | 0.318 |
| Log-rank trend p= | 0.0001 | 0.0023 | 0.355 |

### Example 4: Clinical trial to determine effect of ACTH(1-17) intrathecal injection in scores of survival rate and functional rating scale

Study type: The purpose of this Phase I clinical trial is to asses the safety of intrathecal injection of ACTH (1-17) in the treatment of Amyotrophic Lateral Sclerosis patients.

Study Design: Treatment, Non-Randomized, Open Label, Uncontrolled, Single Group Assignment, Safety/Efficacy Study.

### Primary Outcome Measures:

Survival rate [Time Frame: Every 3 months]
Functional rating scale [Time Frame: Every 3 months]

### Secondary Outcome Measures:

ALS-FRS, MRC and Norris scales [Time Frame: Every 3 months]
Adverse events [Time Frame: Every 3 months]
Estimated Enrollment: 50

### Eligibility:

Ages Eligible for Study: 20 Years to 65 Years
Genders Eligible for Study: Both
Accepts Healthy Volunteers: No

### Inclusion Criteria:

Diagnose established following the World Federation of Neurology criteria; More than 6 and less than 36 months of evolution of the disease; Medullar onset of the disease; More than 20 and less than 65 years old; Forced Vital Capacity equal or superior to 50%; Total time of oxygen saturation <90% inferior to 2% of the sleeping time; Signed informed consent.

### Exclusion Criteria:

Neurological or psychiatric concomitant disease; Need of parenteral or enteral nutrition through percutaneous endoscopic gastrostomy or nasogastric tube; Concomitant systemic disease; Treatment with corticosteroids, immunoglobulins or immunosuppressors during the last 12 months; Inclusion in other clinical trials; inability to understand the informed consent.

### Example 5: Clinical trial to determine effect of ACTH intramuscular injection in scores of survival rate and functional rating scale

Study type: This is a Phase II interventional study to investigate the efficacy and confirm the safety of a pulsed dosing regimen of intramuscular ACTHAR® gel in patients with Amyotrophic Lateral Sclerosis (ALS) by assessing changes in scores of survival rate and functional rating scale.

Study Design: Treatment, Randomized, Double Blind (Subject, Investigator), Placebo Control, Parallel Assignment, Safety/Efficacy Study

### Primary Outcome Measures:

Survival rate [Time Frame: Every 3 months]
Functional rating scale [Time Frame: Every 3 months]

### Secondary Outcome Measures:

Manual Muscle Test (MMT) [Time Frame: Every 3 months]
Percent-predicted forced vital capacity (% FVC) [Time Frame: Every 3 months]
Adverse events [Time Frame: Every 3 months]
Estimated Enrollment: 360
Intervention Drug: ACTHAR® gel

Intramuscular injection, ACTHAR® gel, a first dose of 20 IU, a second dose of 20 IU in the same week, then 40 IU twice a week for one week, then 40 IU every other month for three months, for 3.5 years.
Placebo Comparator Drug: Placebo

Intramuscular injection, ACTHAR® gel, twice a week for two weeks, then every other month for three months, for 3.5 years.

### Eligibility

Ages Eligible for Study: 20 Years and older
Genders Eligible for Study: Both
Accepts Healthy Volunteers: No

### Inclusion Criteria:

Patients who are able to submit written informed consent. If patients are duly capable of study consent but are unable to sign (or affix a seal) by themselves due to aggravation of disease condition, written informed consent can be obtained from a legally authorized representative who can sign on behalf of the patients after confirming the patients' agreement to study participation. Patients who are aged 20 years or older at the time of obtaining informed consent. Patients who have clinically definite ALS, clinically probable ALS, or clinically probable-laboratory supported ALS as specified in the revised El Escorial Airlie House diagnostic criteria. Patients who are at stage 1 or 2 of the severity criteria for ALS. Patients within 3-year elapsed time period from disease onset at the start of observation period. Patients who can visit study site for out-patient treatment.

### Exclusion Criteria:

Patients who underwent tracheostomy. Patients who experienced non-invasive positive pressure ventilation. Patients whose percent-predicted forced vital capacity (%FVC) is >=60%. Patients with multiple disturbances of conduction detected by nerve conduction test. Patients with neurological symptom(s) due to vitamin B12 deficiency. Patients who initiated newly introduced riluzole therapy after starting the observation period. Or those who received dose escalation or resumed administration of riluzole therapy after previous down titration or discontinuation. Patients with cognitive impairment. Pregnant women or women with a possibility of becoming pregnant. Patients with severe disease in the renal, cardiovascular, hematological, or hepatic system (severe disease will be judged referring to "Ministry of Health, Labor and Welfare" (MHLW) Drug Safety Dept. Notification No. 80, Drug Safety Classification Criteria for Severity of Adverse Drug Reaction by Medicinal Products, Grade 3."). Patients with malignant tumor. Patients who participated in another clinical study within 12 weeks before starting the observation period. Patients with present illness or history of drug allergy or severe allergic disease (anaphylactic shock). Patients who are judged to be ineligible for study entry by the investigator or subinvestigator.

### Example 6: Pharmaceutical Compositions

The compositions exemplified below fall within the scope of the present invention when an ACTH₁₋₃₉ peptide is used in delaying progression of ALS.

### Example 6a: Parenteral Composition

To prepare a parenteral pharmaceutical composition suitable for administration by intrathecal or intramuscular or intravenous or subcutaneous injection, 100 mg of a water-soluble salt of ACTH peptide or fragment, analog, complex or aggregate thereof, or any combination thereof, described herein, is dissolved in DMSO and then mixed with 10 mL of 0.9% sterile saline. A preservative and/or a stabilizer is optionally added to the mixture. The mixture is incorporated into a dosage unit form suitable for administration by injection.

### Example 6b: Inhalation Composition

To prepare a pharmaceutical composition for inhalation delivery, 20 mg of ACTH peptide or fragment, analog, complex or aggregate thereof, or any combination thereof, is mixed with 50 mg of anhydrous citric acid and 100 mL of 0.9% sodium chloride solution. The mixture is incorporated into an inhalation delivery unit, such as a nebulizer, which is suitable for inhalation administration.

### Example 6c: Rectal Gel Composition

To prepare a pharmaceutical composition for rectal delivery, 100 mg of ACTH peptide or fragment, analog, complex or aggregate thereof, or any combination thereof, is mixed with 2.5 g of methylcelluose (1500 mPa), 100 mg of methylparapen, 5 g of glycerin and 100 mL of purified water. The resulting gel mixture is then incorporated into rectal delivery units, such as syringes, which are suitable for rectal administration.

### Example 6d: Topical Gel Composition

To prepare a pharmaceutical topical gel composition, 100 mg of ACTH peptide or fragment, analog, complex or aggregate thereof, or any combination thereof, is mixed with 1.75 g of hydroxypropyl celluose, 10 mL of propylene glycol, 10 mL of isopropyl myristate and 100 mL of purified alcohol USP. The resulting gel mixture is then incorporated into containers, such as tubes, which are suitable for topicl administration.

### Example 6e: Oral Composition

To prepare a pharmaceutical composition for oral delivery, 100 mg of ACTH peptide or fragment, analog, complex or aggregate thereof, or any combination thereof, or a prodrug thereof, is mixed with 750 mg of starch. The mixture is incorporated into an oral dosage unit, such as a hard gelatin capsule, which is suitable for oral administration.

### Example 6f: Nasal spray solution

To prepare a pharmaceutical nasal spray solution, 10 g of ACTH peptide or fragment, analog, complex or aggregate thereof, or any combination thereof, is mixed with 30 mL of a 0.05M phosphate buffer solution (pH 4.4). The solution is placed in a nasal administrator designed to deliver 100 µl of spray for each application.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. Adrenocorticotropic hormone (ACTH) peptide (ACTH₁₋₃₉ peptide) for use in delaying progression of Amyotrophic Lateral Sclerosis (ALS) in an individual suspected of having, or predisposed to Amyotrophic Lateral Sclerosis (ALS), wherein the ACTH peptide is to be administered as a first dose and one or more subsequent doses.

2. The ACTH peptide for use as claimed in claim 1, for administration in early stage ALS upon onset of muscle weakness in the limbs and/or slurred and nasal speech.

3. The ACTH peptide for use as claimed in claim 1, wherein the ACTH peptide is to be administered upon detection of a mutation in the SOD1 gene.

4. The ACTH peptide for use as claimed in claim 1, wherein the first dose of ACTH peptide comprises a dose between 10 IU and 150 IU, and the one or more subsequent doses of ACTH peptide is administered every day, every 2 days, every 5 days, every week, every two weeks, every three weeks, every month, every two months, or any combination thereof.

5. The ACTH peptide for use as claimed in claim 1, wherein the first dose of ACTH peptide comprises a dose between 10 IU and 150 IU, and the one or more subsequent doses of ACTH peptide are between 20% - 80% of the first dose.

6. The ACTH peptide for use as claimed in claim 1, wherein the doses of ACTH peptide are to be administered every 2 days.

7. The ACTH peptide, for use as claimed in claim 1, wherein the doses of ACTH peptide are to be administered every 7 days.

8. The ACTH peptide for use as claimed in any of claims 1-7, wherein the ACTH peptide is a porcine, human, or recombinant ACTH peptide.

9. The ACTH peptide for use as claimed in any of claims 1-7, wherein the ACTH peptide is of the formula:

H -Ser-Tyr-Ser-Met-Glu-His-Phe-Arg-Trp-Gly –
1 2 3 4 5 6 7 8 9 10
Lys-Pro-Val-Gly-Lys-Lys-Arg-Arg-Pro-Val –
11 12 13 14 15 16 17 18 19 20
Lys-Val-Tyr-Pro-Asp-Gly-Ala-Glu-Asp-Gln –
21 22 23 24 25 26 27 28 29 30
Leu-Ala-Glu-Ala-Phe-Pro-Leu-Glu-Phe-OH
31 32 33 34 35 36 37 38 39.

10. The ACTH peptide for use as claimed in any of claims 1-7 together with an ACTH fragment of the formula:

H -Ser-Tyr-Ser-Met-Glu-His-Phe-Arg-Trp-Gly –
1 2 3 4 5 6 7 8 9 10
Lys-Pro-Val-Gly-Lys-Lys-Arg-Arg-Pro-Val –
11 12 13 14 15 16 17 18 19 20
Lys-Val-Tyr-Pro
21 22 23 24.

11. The ACTH peptide for use as claimed in any one of claims 1-7, for administration either as a modified release formulation or as an immediate release formulation.

12. The ACTH peptide for use as claimed in any one of claims 1-7 for subcutaneous administration.

13. The ACTH peptide for use as claimed in any one of claims 1-7 for intramuscular administration.

14. The adrenocorticotropic hormone (ACTH) peptide for the use of any one of claims 1-13, further comprising administration of a second therapeutic agent selected from riluzole, ceftriaxone, methylcobalamine, Aeolus 10150, edaravone, hepatocyte growth factor (HGF), insulin growth factor (IGF), Atorvastatin, Lithium carbonate, Avanier 07-ACR-123 (Zenvia®), SB-509, Talampanel, Thalidomide, Arimoclomol, Olanzapine, KNS-760704, memantine, tamoxifen, ONO-2506P0, MCI-186, pioglitazone, ALS-357, creatine monohydrate, TCH346, Botulinum toxin type B, tauroursodeoxycholic acid, Dronabinol, coenzyme Q10, YAM80, Olesoxime, escitalopram (Lexapro®), sodium phenylbutyrate, ISIS 333611, granulocyte stimulating factor, neuronal growth factor (NGF), brain-derived neurotrophic factor (BDNF), neurotrophin 3 (NT3), basic fibroblast growth factor (bFGF), R(+) pramipexole dihydrochloride monohydrate, Sodium Valproate, AVP-923, sNN0029, Antithymocyte globulin, cyclosporin, corticosteroids, modafinil, or anti-CD4OL, wherein the second therapeutic agent is to be administered sequentially or simultaneously.

## Patentansprüche

1. Adrenocorticotropes Hormon (ACTH)-Peptid (ACTH₁₋₃₉-Peptid) zur Verwendung bei der Verzögerung des Fortschreitens der Amyotrophen Lateralsklerose (ALS) bei einem Individuum, bei dem der Verdacht besteht, dass es eine solche Krankheit hat, oder prädisponiert für Amyotrophe Lateralsklerose (ALS) ist, wobei das ACTH-Peptid als erste Dosis und eine oder mehrere nachfolgende Dosen verabreicht werden soll.

2. ACTH-Peptid zur Verwendung wie in Anspruch 1 beansprucht zur Verabreichung in einem frühen Stadium der ALS bei Auftreten von Muskelschwäche in den Gliedmaßen und/oder undeutlicher und nasaler Sprache.

3. ACTH-Peptid zur Verwendung wie in Anspruch 1 beansprucht, wobei das ACTH-Peptid bei Nachweis einer Mutation im SOD1-Gen verabreicht werden soll.

4. ACTH-Peptid zur Verwendung wie in Anspruch 1 beansprucht, wobei die erste Dosis des ACTH-Peptids eine Dosis zwischen 10 IE und 150 IE umfasst und die eine oder mehrere aufeinanderfolgende Dosen des ACTH-Peptids jeden Tag, alle 2 Tage, alle 5 Tage, jede Woche, alle zwei Wochen, alle drei Wochen, jeden Monat, alle zwei Monate oder jede Kombination davon verabreicht werden.

5. ACTH-Peptid zur Verwendung wie in Anspruch 1 beansprucht, wobei die erste Dosis des ACTH-Peptids eine Dosis zwischen 10 IE und 150 IE umfasst und die eine oder mehrere aufeinanderfolgende Dosen des ACTH-Peptids zwischen 20 % und 80 % der ersten Dosis liegen.

6. ACTH-Peptid zur Verwendung wie in Anspruch 1 beansprucht, wobei die Dosen von ACTH-Peptid alle 2 Tage verabreicht werden sollen.

7. ACTH-Peptid zur Verwendung wie in Anspruch 1 beansprucht, wobei die Dosen des ACTH-Peptids alle 7 Tage verabreicht werden sollen.

8. ACTH-Peptid zur Verwendung wie in einem der Ansprüche 1-7 beansprucht, wobei das ACTH-Peptid ein Schweine-, Human- oder rekombinantes ACTH-Peptid ist.

9. ACTH-Peptid zur Verwendung wie in einem der Ansprüche 1-7 beansprucht, wobei das ACTH-Peptid der folgenden Formel entspricht:

H -Ser-Tyr-Ser-Met-Glu-His-Phe-Arg-Trp-Gly –
1 2 3 4 5 6 7 8 9 10
Lys-Pro-Val-Gly-Lys-Lys-Arg-Arg-Pro-Val –
11 12 13 14 15 16 17 18 19 20
Lys-Val-Tyr-Pro-Asp-Gly-Ala-Glu-Asp-Gln –
21 22 23 24 25 26 27 28 29 30
Leu-Ala-Glu-Ala-Phe-Pro-Leu-Glu-Phe-OH
31 32 33 34 35 36 37 38 39.

10. ACTH-Peptid zur Verwendung wie in einem der Ansprüche 1-7 beansprucht zusammen mit einem ACTH-Fragment der folgenden Formel:

H -Ser-Tyr-Ser-Met-Glu-His-Phe-Arg-Trp-Gly –
1 2 3 4 5 6 7 8 9 10
Lys-Pro-Val-Gly-Lys-Lys-Arg-Arg-Pro-Val –
11 12 13 14 15 16 17 18 19 20
Lys-Val-Tyr-Pro
21 22 23 24.

11. ACTH-Peptid zur Verwendung wie in einem der Ansprüche 1-7 beansprucht zur Verabreichung entweder als Formulierung mit modifizierter Freisetzung oder als Formulierung mit sofortiger Freisetzung.

12. ACTH-Peptid zur Verwendung wie in einem der Ansprüche 1-7 beansprucht zur subkutanen Verabreichung.

13. ACTH-Peptid zur Verwendung wie in einem der Ansprüche 1-7 beansprucht zur intramuskulären Verabreichung.

14. Adrenocorticotropes Hormon (ACTH)-Peptid zur Verwendung nach einem der Ansprüche 1-13, ferner umfassend die Verabreichung eines zweiten therapeutischen Mittels, ausgewählt aus Riluzol, Ceftriaxon, Methylcobalamin, Aeolus 10150, Edaravon, Hepatozytenwachstumsfaktor (HGF), Insulin-Wachstumsfaktor (IGF), Atorvastatin, Lithiumcarbonat, Avanier 07-ACR-123 (Zenvia®), SB-509, Talampanel, Thalidomid, Arimoclomol, Olanzapin, KNS-760704, Memantin, Tamoxifen, ONO-2506P0, MCI-186, Pioglitazon, ALS-357, Kreatinmonohydrat, TCH346, Botulinumtoxin Typ B, Tauroursodeoxycholsäure, Dronabinol, Coenzym Q10, YAM80, Olesoxime, Escitalopram (Lexapro®), Natriumphenylbutyrat, ISIS 333611, Granulozytenstimulierender Faktor, neuronaler Wachstumsfaktor (NGF), vom Gehirn stammender neurotropher Faktor (BDNF), Neurotrophin 3 (NT3), basischer Fibroblasten-Wachstumsfaktor (bFGF), R(+) Pramipexoldihydrochloridmonohydrat, Natriumvalproat, AVP-923, sNNN0029, Antithymozytenglobulin, Cyclosporin, Kortikosteroide, Modafinil oder Anti-CD40L, wobei das zweite therapeutische Mittel zeitlich versetzt oder gleichzeitig verabreicht werden soll.

## Revendications

1. Peptide d'hormone corticotrope (ACTH) (peptide ACTH₁₋₃₉) pour une utilisation dans le retard de la progression de la sclérose latérale amyotrophique (SLA) chez un individu suspecté d'avoir, ou prédisposé à la sclérose latérale amyotrophique (SLA), où le peptide d'ACTH est destiné à être administré sous forme d'une première dose et d'une ou plusieurs doses subséquentes.

2. Peptide d'ACTH pour une utilisation selon la revendication 1, pour une administration dans la SLA au stade précoce lors du déclenchement d'une faiblesse musculaire dans les membres et/ou d'une élocution indistincte et nasale.

3. Peptide d'ACTH pour une utilisation selon la revendication 1, où le peptide d'ACTH est destiné à être administré lors de la détection d'une mutation dans le gène SOD1.

4. Peptide d'ACTH pour une utilisation selon la revendication 1, où la première dose de peptide d'ACTH comprend une dose entre 10 UI et 150 UI, et les une ou plusieurs doses subséquentes de peptide d'ACTH sont administrées tous les jours, tous les 2 jours, tous les 5 jours, toutes les semaines, toutes les deux semaines, toutes les trois semaines, tous les mois, tous les deux mois, ou toute combinaison de ceux-ci.

5. Peptide d'ACTH pour une utilisation selon la revendication 1, où la première dose de peptide d'ACTH comprend une dose entre 10 UI et 150 UI, et les une ou plusieurs doses subséquentes de peptide d'ACTH sont entre 20 % - 80 % de la première dose.

6. Peptide d'ACTH pour une utilisation selon la revendication 1, où les doses de peptide d'ACTH sont destinées à être administrées tous les 2 jours.

7. Peptide d'ACTH, pour une utilisation selon la revendication 1, où les doses de peptide d'ACTH sont destinées à être administrées tous les 7 jours.

8. Peptide d'ACTH pour une utilisation selon l'une quelconque des revendications 1 - 7, où le peptide d'ACTH est un peptide d'ACTH porcin, humain ou recombinant.

9. Peptide d'ACTH pour une utilisation selon l'une quelconque des revendications 1 - 7, où le peptide d'ACTH est de formule:

H-Ser-Tyr-Ser-Met-Glu-His-Phe-Arg-Trp-Gly-
1 2 3 4 5 6 7 8 9 10

Lys-Pro-Val-Gly-Lys-Lys-Arg-Arg-Pro-Val-
11 12 13 14 15 16 17 18 19 20
Lys-Val-Tyr-Pro-Asp-Gly-Ala-Glu-Asp-Gln-
21 22 23 24 25 26 27 28 29 30
Leu-Ala-Glu-Ala-Phe-Pro-Leu-Glu-Phe-OH
31 32 33 34 35 36 37 38 39.

10. Peptide d'ACTH pour une utilisation selon l'une quelconque des revendications 1 - 7 ensemble avec un fragment d'ACTH de formule:

H-Ser-Tyr-Ser-Met-Glu-His-Phe-Arg-Trp-Gly-
1 2 3 4 5 6 7 8 9 10
Lys-Pro-Val-Gly-Lys-Lys-Arg-Arg-Pro-Val-
11 12 13 14 15 16 17 18 19 20
Lys-Val-Tyr-Pro
21 22 23 24.

11. Peptide d'ACTH pour une utilisation selon l'une quelconque des revendications 1 - 7, pour une administration sous forme d'une formulation à libération modifiée ou sous forme d'une formulation à libération immédiate.

12. Peptide d'ACTH pour une utilisation selon l'une quelconque des revendications 1 - 7 pour une administration sous-cutanée.

13. Peptide d'ACTH pour une utilisation selon l'une quelconque des revendications 1 - 7 pour une administration intramusculaire.

14. Peptide d'hormone corticotrope (ACTH) pour l'utilisation selon l'une quelconque des revendications 1 - 13, comprenant en outre l'administration d'un second agent thérapeutique choisi parmi le riluzole, la ceftriaxone, la méthylcobalamine, Aeolus 10150, l'édaravone, le facteur de croissance des hépatocytes (HGF), le facteur de croissance apparenté à l'insuline (IGF), l'atorvastatine, le carbonate de lithium, Avanier 07-ACR-123 (Zenvia®), SB-509, le talampanel, la thalidomide, l'arimoclomol, l'olanzapine, KNS-760704, la mémantine, le tamoxifène, ONO-2506P0, MCI-186, la pioglitazone, ALS-357, la créatine monohydratée, TCH346, la toxine botulique de type B, l'acide tauroursodésoxycholique, le dronabinol, le coenzyme Q10, YAM80, l'olésoxime, l'escitalopram (Lexapro®), le phénylburyrate de sodium, ISIS 333611, le facteur stimulant les granulocytes, le facteur de croissance neuronal (NGF), le facteur neurotrophique dérivé du cerveau (BDNF), la neurotrophine 3 (NT3), le facteur de croissance des fibroblastes basique (bFGF), le dichlorhydrate de R(+) pramipexole monohydraté, le valproate de sodium, AVP-923, sNN0029, la globuline antithymocyte, la cyclosporine, les corticostéroïdes, le modafinil ou l'anti-CD4OL, où le second agent thérapeutique est destiné à être administré successivement ou simultanément.
